# EUROPEAN PATENT SPECIFICATION

(11) **EP 2 455 486 B1**
(45) Date of publication and mention of the grant of the patent: **11.01.2017**
(21) Application number: 10799873.4
(22) Date of filing: 14.07.2010
(51) Int. Cl.: C12Q 1/68, C12N 15/09

(54) **METHOD FOR SCREENING FOR DIET PROVIDING PRODUCTION OF MILK HAVING IMMUNOREGULATORY ACTION**
SCREENING-VERFAHREN ZUR HERSTELLUNG DIÄTGEEIGNETER MILCH MIT IMMUNREGULATORISCHER WIRKUNG
PROCÉDÉ DE CRIBLAGE D'ALIMENT POUR ANIMAUX PERMETTANT LA PRODUCTION DE LAIT AYANT UN EFFET IMMUNOMODULATEUR

(30) Priority: 14.07.2009 JP 2009165991
(43) Date of publication of application: 23.05.2012
(73) Proprietor: Morinaga Milk Industry Co., Ltd., Minato-ku Tokyo 108-8384 (JP)
(72) Inventor: OCHIYA, Takahiro, Tokyo 104-0045 (JP); KOSAKA, Nobuyoshi, Tokyo 165-0026 (JP); SEKINE, Kazunori, Zama-shi Kanagawa 252-8583 (JP); IZUMI, Hirohisa, Zama-shi Kanagawa 252-8583 (JP)
(74) Representative: Henderson, Helen Lee
(86) International application number: PCT/JP2010/061926
(87) International publication number: WO 2011/007815

(56) References cited:
- WO-A1-2008/147974
- A H LUND: "miR-10 in development and cancer", CELL DEATH AND DIFFERENTIATION, vol. 17, no. 2, 22 May 2009 (2009-05-22), pages 209-214, XP055089333, ISSN: 1350-9047, DOI: 10.1038/cdd.2009.58
- CLOTILDE THÉRY ET AL: "Membrane vesicles as conveyors of immune responses", NATURE REVIEWS IMMUNOLOGY, vol. 9, no. 8, 5 June 2009 (2009-06-05), pages 581-593, XP55018445, ISSN: 1474-1733, DOI: 10.1038/nri2567
- ANGELA DOLGANIUC ET AL: "MicroRNA Expression Profile in Lieber-DeCarli Diet-Induced Alcoholic and Methionine Choline Deficient Diet-Induced Nonalcoholic Steatohepatitis Models in Mice", ALCOHOLISM: CLINICAL & EXPERIMENTAL RESEARCH, vol. 33, no. 10, 1 October 2009 (2009-10-01), pages 1704-1710, XP055089337, ISSN: 0145-6008, DOI: 10.1111/j.1530-0277.2009.01007.x
- QI ZHOU ET AL: "Immune-related MicroRNAs are Abundant in Breast Milk Exosomes", INTERNATIONAL JOURNAL OF BIOLOGICAL SCIENCES, vol. 8092311231129, 29 November 2011 (2011-11-29), pages 118-123, XP055089341, ISSN: 1449-2288, DOI: 10.7150/ijbs.8.118
- GU, Z. ET AL.: 'Identification and characterization of microRNAs from the bovine adipose tissue and mammary gland.' FEES LETT. vol. 581, no. 5, 2007, pages 981 - 988
- ADMYRE, C. ET AL.: 'Exosomes with immune modulatory features are present in human breast milk.' J. IMMUNOL. vol. 179, no. 3, 2007, pages 1969 - 1978
- HUNTER, M.P. ET AL.: 'Detection of microRNA expression in human peripheral blood microvesicles.' PLOS ONE vol. 3, no. 11, 2008, page E3694, XP008149486
- VALADI, H. ET AL.: 'Exosome-mediated transfer of mRNAs and microRNAs is a novel mechanism of genetic exchange between cells.' NAT. CELL BIOL. vol. 9, no. 6, 2007, pages 654 - 659
- KOSAKA. N. ET AL.: 'microRNA as a new immune- regulatory agent in breast milk.' SILENCE vol. 1, 01 March 2010, page 7
- HATA, T. ET AL.: 'Isolation of bovine milk- derived microvesicles carrying mRNAs and microRNAs.' BIOCHEM. BIOPHYS. RES. COMMUN. vol. 396, no. 2, 28 May 2010, pages 528 - 533
- Laura M'rabet ET AL: "Breast-Feeding and Its Role in Early Development of the Immune System in Infants: Consequences for Health Later in Life", The Journal of Nutrition, vol. 138, 1 January 2008 (2008-01-01), pages 1782S-1790S, XP055131328,
- Henk K A Visser: "Influence of Diet on Infection and Allergy in Infants Dietary Influences on Infection and Allergy in Infants: Introduction", The Journal of Nutrition, vol. 138, 1 January 2008 (2008-01-01), pages 1768S-1769S, XP055131342,
- LAKKARAJU A ET AL: "Itinerant exosomes: emerging roles in cell and tissue polarity", TRENDS IN CELL BIOLOGY, ELSEVIER SCIENCE LTD, XX, vol. 18, no. 5, 1 May 2008 (2008-05-01), pages 199-209, XP022636891, ISSN: 0962-8924, DOI: 10.1016/J.TCB.2008.03.002 [retrieved on 2008-04-07]
- VALADI, H. ET AL.: NAT. CELL BIOL., vol. 9, 2007, pages 654-659,
- XI CHEN1 ET AL: "Identification and characterization of microRNAs in raw milk during different periods of lactation, commercial fluid, and powdered milk products", CELL RESEARCH - XIBAO YANJIU, NATURE PUBLISHING GROUP, GB, CN, vol. 20, no. 10, 1 October 2010 (2010-10-01), pages 1128-1137, XP002680648, ISSN: 1001-0602, DOI: 10.1038/CR.2010.80 [retrieved on 2010-06-15]

## Description

### Technical Field

The present invention relates to a method for screening for a diet providing production of milk having an immunoregulatory action, which is useful in the fields of foodstuff, animal feed, and so forth.

### Background Art

Immunity of living organisms essentially functions for the purpose of "defense" against external attacks. For example, phylaxis and elimination of cancer cells correspond to the "defense", and enhancement of the immunity effectively operates in such a case.

On the other hand, overresponse of the immunity, i.e., "hyperimmunity", may adversely affect living organisms. Examples thereof include allergic responses, autoimmune diseases, chronic inflammations, and so forth. It is known that, in such a case, symptoms are improved by suppressing production of inflammatory cytokines such as IL-6, TNF-α and IL-1.

Further, it is becoming clear that immunostimulating actions functioning for the purpose of "defense" against external attacks, and immunosuppressive actions functioning for suppressing allergic responses, autoimmune diseases, chronic inflammations etc. induced by hyperimmunoreaction are regulated by microRNA (henceforth also referred to as "miRNA").

After a miRNA is transcribed from genome, it undergoes two times of cleavage and becomes a non-coding small RNA of about 22 bases. It is known that, as a function thereof, it binds to a 3'-untranslated region of target mRNAs in a sequence-complementary manner to suppress translation of the target mRNAs. One kind of miRNA inhibits translation of a plurality of kinds of mRNAs in a cell to regulate various functions of the cell. Many reports have been made especially on relations thereof with development and evolution of cancers, and relation between miRNA and diseases attracts attention.

For example, as for miR-181, it has been reported that it is involved in development of B cells, activation of T cells, and development of immunity (Non-patent documents 1 to 3).

As for miR-155, it is known that it is involved in development of immunity through activation of the innate immunity (Non-patent documents 1 and 4) and regulation of differentiation and functions of T cells and B cells (Non-patent documents 1 and 5), and it is involved in antiallergy and anti-inflammation through regulation of Th1/Th2 balance (Non-patent documents 1 and 6) and maintenance of the functions of regulatory T cells, which suppress hyperimmunoreactions (Non-patent document 7).

miR-17 and miR-92 cooperate to regulate differentiation and development of B cells and T cells and thereby participate in development of immunity (Non-patent documents 1, 8 and 9).

It is known that miR-223 participates in phylaxis by controlling proliferation and activation of neutrophils (Non-patent documents 1 and 10), miR-150 participates in phylaxis by suppressing differentiation of B cells (Non-patent document 1 and 11), and let-7i participates in phylaxis by controlling TLR4 expression in cholangiocytes (Non-patent document 12).

It is known that miR-125 participates in anti-inflammation by suppressing production of TNF-α (Non-patent documents 1 and 13).

It is known that miR-146 participates in phylaxis by negatively regulating the innate immunity (Non-patent documents 1 and 14), and participates in antiallergy by controlling Th1/Th2 balance (Non-patent document 15).

It has recently been reported that miRNAs which function in cells as translation regulatory molecules are present in a lipid bilayer called exosome, and are secreted out of the cells (Non-patent document 16).
Since it has also been confirmed that secreted miRNAs are incorporated into other cells, presence of intercellular actions by means of miRNA has been presented. Further, exosomes are known to be present in various kinds of human body fluids. In particular, presence of miRNAs in human plasma and serum has already been reported, and a possibility of use thereof as a biomarker of prostate cancer or uterine cancer has been suggested (Non-patent document 17).

Body fluids containing exosomes include, besides plasma and serum, saliva, urine, amniotic fluid and breast milk (Non-patent document 17). Among these, breast milk is a body fluid produced by mammals in a specific period, and responsible for transfer of substances between individuals, i.e., from a mother to a child. Moreover, breast milk not only supplements nutrients to a child, but also gives immune substances acquired by a mother to a child.

Admyre et al. (non-patent document 18) is concerned with methods investigating immunoactivity of breastmilk and discloses a method of isolating exosomes from colostrum and mature breast milk, and association with immune responses.

Breast milk contains secretory IgA, lactoferrin, lysozyme, cytokines, and so forth, and it is considered that it protects infants from infection, and promotes development of infant's immunity (Non-patent document 19). Actually, it is known that children grown up on breast milk involve a lower risk of infection in the bronchi or intestinal tract as compared to children not grown in such a manner. Breast milk contains IgA, lactoferrin, glycoproteins, glycolipids etc. which show antibacterial activities, as well as cytokines which regulate immunocytes. However, the objects analyzed in the researches to date are mainly proteins contained in breast milk, and although there are reports on nucleic acids contained in breast milk, researches on nucleic acids contained in breast milk and having specific sequences have not been deported.

Moreover, it is also known that development of mammary glandular cells controlled by expression of cyclooxygenase 2 is regulated by miR-101a (Non-patent document 20). However, it is not suggested that miRNAs exist in milk.

In addition, after the priority date of this application, it has been reported that microRNAs are present in microvesicles derived from bovine milk (Non-patent document 21), and microRNAs are identified in fresh milk of bovines of different lactation periods, commercial liquid milk and dried milk (Non-patent document 22).

### Prior art references

### [Non-patent documents]

Non-patent document 1: Lindsay, M.A., Trends Immunol, 29:343-351, 2008
Non-patent document 2: Li, Qi-Jing et al., Cell, 129:147-161, 2007
Non-patent document 3: Chen, Chang-zheng et al., Science, 303:83-86, 2004
Non-patent document 4: O'Connel, R.M. et al., PNAS, 104 (5):1604-1609, 2007
Non-patent document 5: Vigorito, E. et al., Immunity, 27:847-859, 2007
Non-patent document 6: Rodriguez, A. et al., Science, 316:608-611, 2007
Non-patent document 7: Kohlhaas, S. et al., J. Immunol., 182:2578-2582, 2009
Non-patent document 8: Koralov, S.B. et al., Cell, 132:860-874, 2008
Non-patent document 9: Xiao, C. et al., Nat. Immunol., 9:405-414, 2008
Non-patent document 10: Jonathan, B. et al., Nature, 451:1125-1129, 2008
Non-patent document 11: Zhou, B. et al., PNAS, 104 (17):7080-7085, 2007
Non-patent document 12: Chen, Xian-Ming et al., J. Biol. Chem., 282 (39):28929-28938, 2007
Non-patent document 13: Tili, E. et al., J. Immunol., 179:5082-5089, 2007
Non-patent document 14: Taganov, K.D. et al., PNAS, 103 (33):12481-12486, 2006
Non-patent document 15: Monticelli, S. et al., Genome Biol., 6, R71, 2005
Non-patent document 16: Valadi, H. et al., Nat. Cell Biol., 9:654-659, 2007
Non-patent document 17: Gilad, S. et al., PLoS One, 3 (9):e3148, 2008
Non-patent document 18: Admyre, C., J. Immunol., 179:1969-1978, 2007
Non-patent document 19: Goldman, A.S., Breastfeed Med., 2 (4):195-204, 2007
Non-patent document 20: Tanaka, T. et al., Differentiation, 77:181-187, 2009
Non-patent document 21: Hata, T. et al., Biochem. Biophys. Res. Commun., 396 (2):528-533, 2010
Non-patent document 22: Chen, X. et al., Cell Research, (2010):1-10

### Summary of the Invention

### Object to be Achieved by the Invention

An object of the present invention is to provide a method for screening for a diet providing production of milk having an immunoregulatory action, a novel foodstuff having an immunoregulatory action, and a method for producing it.

### Means for Achieving the Object

The inventors of the present invention conducted researches with paying attention to the fact that breast milk affected maturation of infant's immune system. As a result, they found that immunity-related miRNAs are highly expressed in breast milk, and accomplished the present invention.

The present invention thus provides a method for screening for a diet or a substance providing production of breast milk having an immunostimulating action, which comprises:
comparing microRNA profiles in milk of rat observed before and after ingestion of a diet or a substance, and when amount of one or more kinds of microRNA in the milk observed after the ingestion of the diet or the substance is higher than that observed before the ingestion, judging that the diet or the substance increases the amount of the microRNA in the milk,
wherein the microRNA is selected from the group consisting of miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p and miR-542-3p.

In a preferred embodiment the aforementioned method, comprises comparing microRNA profiles in the milk of the rat observed before and after ingestion of the diet or the substance with microRNA profiles in serum or plasma of the rat observed before and after ingestion of the diet or the substance, respectively, and
when the amount of microRNA contained in both the milk and the serum or plasma is increased by ingestion of the diet or substance, judging that the diet or the substance increases the amount of the microRNA in the milk.

In another embodiment of the aforementioned method, the present invention provides a method for screening for a diet or a substance providing production of breast milk having an immunostimulating action, which comprises:
comparing microRNA profiles in milk of bovine observed before and after ingestion of a diet or a substance, and when amount of one or more kinds of microRNA in the milk observed after the ingestion of the diet or the substance is higher than that observed before the ingestion, judging that the diet or the substance increases the amount of the microRNA in the milk,
wherein the microRNA is selected from the group consisting of miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d and let-7i.

A preferred embodiment of the aforementioned method comprises comparing microRNA profiles in the milk of the bovine observed before and after ingestion of the diet or the substance with microRNA profiles in serum or plasma of the bovine observed before and after ingestion of the diet or the substance, respectively, and
when the amount of microRNA contained in both the milk and the serum or plasma is increased by ingestion of the diet or substance, judging that the diet or the substance increases the amount of the microRNA in the milk.

The present invention also provides a method for producing milk or dairy products having an immunostimulating action, which comprises:
the step of giving *Bifidobacterium* bacteria to rat, and
the step of collecting milk from the rat, wherein the collected milk comprises microRNA selected from the group consisting of miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p and miR-542-3p.

### Brief Description of the Drawings

Fig. 1 shows results of detection of miRNAs in human breast milk obtained by microarray analysis.
Fig. 2 shows comparison of miR-181a levels in breast milk for first six months after birth and six months thereafter. hsa represents human, and cel represents a nematode (*Caenorhabditis elegans*) (the same shall apply to the following drawings).
Fig. 3 shows comparison of miR-155, miR-17, and miR-92 levels in breast milk for first six months after birth and six months thereafter.
Fig. 4 shows comparison of immunity-related miRNA levels in human breast milk and serum.
Fig. 5 shows comparison of miRNA levels observed before and after freeze-thaw.
Fig. 6 shows comparison of miRNA levels observed before and after storage at low pH (pH 1).
Fig. 7 shows comparison of miRNA levels observed after RNases treatment and without RNases treatment.

### Embodiments for Carrying out the Invention

The method of the present invention is a method for screening for a diet or a substance providing production of breast milk having an immunostimulatory action, which comprises identifying a diet or a substance that increases amount of microRNA present in milk of a mammal on the basis of correlation of microRNA profiles in the milk and a diet ingested by the mammal or a substance contained in the diet.

The immunoregulatory action is an immunostimulating action, and when the amount of the microRNA increases, it is judged that the diet or substance provides production of breast milk having an immunostimulating action.

The present invention is based on a concept that an immunoregulatory action is expected to be obtained by oral administration of miRNA, because of the novel finding that miRNAs are contained in milk, and the fact that miRNAs can stably exist even under acidic conditions in the stomach, and breast milk promotes development of immunity in infants ingesting the breast milk (for example, Breastfeed Med., 2(4):195-204, 2007). And, on the basis of a prediction that a miRNA profile in milk is affected by diet, it was thought to identify a diet or an active ingredient contained in it that could increase or decrease amount of miRNA present in milk.

The immunoregulatory action defined for the screening method, milk, dairy product, and so forth of the present invention is an action of enhancing immunopotentiating action, which functions for the purpose of "defense" against external attacks (immunostimulating action).

The terms "immunostimulating action" and "immunosuppressive action" are used in a relative meaning.

When an immunopotentiating action usually observed for breast milk of a certain mammal is enhanced after ingestion of the diet or substance, the breast milk has an immunostimulating action. When the immunopotentiating action observed after ingestion of the diet or substance by a mammal is enhanced as compared to that observed before the ingestion, the breast milk of the mammal has an immunostimulating action.

The correlation of miRNA profiles in milk of a mammal and a diet ingested by the mammal or a substance contained in the diet can be investigated, for example, as follows.

Milk is collected from a mammal that ingested a diet, and a miRNA profile in the milk is examined.

The mammal is bovine or rat.

In the present invention, the miRNA profile consists of type and amount of miRNA. The miRNA may consist of one kind of miRNA, or two or more kinds of miRNAs. As described above, it
is known that breast milk promotes development of immunity in infants who ingest it (for example, Breastfeed Med., 2(4):195-204, 2007). Moreover, it has been reported that many components considered to be important to the immune system of infants (including animal infants) are generally contained in colostrum (J. Anim. Sci., 2009, 87:(Suppl.1): 3-9). Therefore, it is suggested that the aforementioned miRNAs of which presence in milk is confirmed are involved in immune functions.

Moreover, among the miRNAs mentioned above, particularly preferred are miRNAs of which presence was confirmed in colostrum of both rat and bovine.

Certain miRNAs have subtypes, and for example, 2 to 4 kinds of subtypes are known for each of miR-181, miR-92, miR-125, miR-146, and so forth, such as miR-181a, miR-181b, miR-181c, miR-181d, miR-92a, miR-92b, miR-125a, miR-125a-3P, miR-125a-5P, miR-125b, miR-146a, miR-146b, miR-146b-3P and miR-146b-5P, respectively. Certain other miRNAs also have subtypes, and in the present invention, the miRNA may be any of such subtypes. Examples of the subtypes include those of which presence in milk was confirmed in the examples described later (refer to Examples 1, 3, 4 and 5).

The nucleotide sequences of human miR-155 precursor, hsa-mir-155 (MI0000681), and the active site thereof, hsa-miR-155 (MIMAT0009241), are shown in SEQ ID NOS: 1 and 2, respectively. Shown in the parentheses are accession numbers in a miRNA database (miRBase::Sequences, http://microrna.sanger.ac.uk/sequences/index.shtml) (the same shall apply to the following descriptions).

The nucleotide sequences of bovine miR-155 precursor, bta-miR-155 (MI0009752), and the active site thereof, bta-miR-155 (MIMAT0000646), are shown in SEQ ID NOS: 3 and 4, respectively.

The nucleotide sequences of human miR-181a precursors, hsa-mir-181a-1 (MI0000289), and hsa-mir-181a-2 (MI0000269), and the active site thereof, hsa-miR-181a (MIMAT0000256), are shown in SEQ ID NOS: 5, 6 and 7, respectively.

The nucleotide sequences of human miR-181b precursors, hsa-mir-181b-1 (MI0000270), and hsa-mir-181b-2 (MI0000683), and the active site thereof, hsa-miR-181b (MIMAT0000257), are shown in SEQ ID NOS: 8, 9 and 10, respectively.

The nucleotide sequences of bovine miR-181a precursors, bta-mir-181a (MI0004757), and bta-mir-181a-1 (MI0010484), and the active site thereof, bta-miR-181a (MIMAT0003543), are shown in SEQ ID NOS: 11, 12 and 13, respectively.

The nucleotide sequences of bovine miR-181b precursors, bta-mir-181b-1 (MI0010485), and bta-mir-181b-2 (MI0005013), and the active site thereof, bta-miR-181b (MIMAT0003793), are shown in SEQ ID NOS: 14, 15 and 16, respectively.

The nucleotide sequences of human miR-223 precursor, hsa-mir-223 (MI0000300), and the active site thereof, hsa-miR-223 (MIMAT0000280), are shown in SEQ ID NOS: 17 and 18, respectively.

The nucleotide sequences of bovine miR-223 precursor, bta-mir-223 (MI0009782), and the active site thereof, bta-miR-223 (MIMAT0009270), are shown in SEQ ID NOS: 19 and 20, respectively.

The nucleotide sequences of human miR-17 precursor, hsa-mir-17 (MI0000071), and the active site thereof, hsa-miR-17 (MIMAT0000070) (also called hsa-miR-17-5p), are shown in SEQ ID NOS: 21 and 22, respectively.

The nucleotide sequences of bovine miR-17 precursor, bta-mir-17 (MI0005031), the active sites thereof, bta-miR-17-5p (MIMAT0003815) and bta-miR-17-3p (MIMAT0003816), are shown in SEQ ID NOS: 23, 24 and 25, respectively.

The nucleotide sequences of human miR-92a precursors, hsa-mir-92a-1 (MI0000093), and hsa-mir-92a-2 (MI0000094), and the active site thereof, hsa-miR-92a (MIMAT0000092), are shown in SEQ ID NOS: 26, 27 and 28, respectively.

The nucleotide sequences of human miR-92b precursor, hsa-mir-92b (MI0003560), and the active site thereof, hsa-miR-92b (MIMAT0003218), are shown in SEQ ID NOS: 29 and 30, respectively.

The nucleotide sequences of bovine miR-92 precursor, bta-mir-92 (MI0005024), and the active site thereof, bta-miR-92 (MIMAT0003808), are shown in SEQ ID NOS: 31 and 32, respectively.

The nucleotide sequences of bovine miR-92a precursor, bta-mir-92a (MI0009905), and the active site thereof, bta-miR-92a (MIMAT0009383), are shown in SEQ ID NOS: 33 and 34, respectively.

The nucleotide sequences of bovine miR-92b precursor, bta-mir-92b (MI0009906), and the active site thereof, bta-miR-92b (MIMAT0009384), are shown in SEQ ID NOS: 35 and 36, respectively.

The nucleotide sequences of human let-7i precursor, hsa-let-7i (MI0000434), and the active site thereof, hsa-let-7i (MIMAT0000415), are shown in SEQ ID NOS: 37 and 38, respectively.

The nucleotide sequences of bovine let-7i precursor, bta-let-7i (MI0005065), and the active site thereof, bta-let-7i (MIMAT0003851), are shown in SEQ ID NOS: 39 and 40, respectively.

The nucleotide sequences of human miR-125a precursor, hsa-mir-125a (MI0000469), and the active sites thereof, hsa-miR-125a-5p (MIMAT0000443) and hsa-miR-125a-3p (MIMAT0004602), are shown in SEQ ID NOS: 41, 42 and 43, respectively.

The nucleotide sequences of human miR-125b precursors, hsa-mir-125b-1 (MI0000446), and hsa-mir-125b-2 (MI0000470), and the active site thereof, hsa-miR-125b (MIMAT0000423), are shown in SEQ ID NOS: 44, 45 and 46, respectively.

The nucleotide sequences of bovine miR-125a precursor, bta-mir-125a (MI0004752), and the active site thereof, bta-miR-125a (MIMAT0003538), are shown in SEQ ID NOS: 47 and 48, respectively.

The nucleotide sequences of bovine miR-125b precursors, bta-mir-125b-1 (MI0004753), and bta-mir-125b-2 (MI0005457), and the active site thereof, bta-miR-125b (MIMAT0003539), are shown in SEQ ID NOS: 49, 50 and 51, respectively.

The nucleotide sequences of human miR-146a precursor, hsa-mir-146a (MI0000477), and the active site thereof, hsa-miR-146a (MIMAT0000449), are shown in SEQ ID NOS: 52 and 53, respectively.

The nucleotide sequences of human miR-146b precursor, hsa-mir-146b (MI0003129), and the active sites thereof, hsa-miR-146b-5p (MIMAT0002809) (also referred to as hsa-miR-146b) and hsa-miR-146b-3p (MIMAT0004766), are shown in SEQ ID NOS: 54, 55 and 56, respectively.

The nucleotide sequences of bovine miR-146a precursor, bta-mir-146a (MI0009746), and the active site thereof, bta-miR-146a (MIMAT0009236), are shown in SEQ ID NOS: 57 and 58, respectively.

The nucleotide sequences of bovine miR-146b precursor, bta-mir-146b (MI0009745), and the active site thereof, bta-miR-146b (MIMAT0009235), are shown in SEQ ID NOS: 59 and 60, respectively.

The nucleotide sequences of human miR-150 precursor, hsa-mir-150 (MI0000479), and the active site thereof, hsa-miR-150 (MIMAT0000451), are shown in SEQ ID NOS: 61 and 62, respectively.

The nucleotide sequences of bovine miR-150 precursor, bta-mir-150 (MI0005058), and the active site thereof, bta-miR-150 (MIMAT0003845), are shown in SEQ ID NOS: 63 and 64, respectively.

In addition to the aforementioned miRNAs, miRNAs of which presence in milk of rat or bovine was confirmed, and miRNAs of other animals corresponding to those miRNAs are shown as Tables 1 to 10.

**Table 1**

| miRNA | Human or animal | Sequence | SEQ ID NO |
|---|---|---|---|
| miR-155 | human | uuaaugcuaaucgugauaggggu | 1 |
| | bovine | uuaaugcuaaucgugauaggggu | 4 |
| miR-17-3p | bovine | acugcagugaaggcacuugu | 25 |
| miR-92 | bovine | uauugcacuugucccggccugu | 32 |
| miR-92b | human | uauugcacucgucccggccucc | 30 |
| | bovine | uauugcacucgucccggccucc | 36 |
| miR-146b-3p | human | ugagaacugaauuccauaggcu | 55 |
| miR-150 | human | ucucccaacccuuguaccagug | 62 |
| | bovine | ucucccaacccuuguaccagugu | 64 |
| miR-17-5p | human | caaagugcuuacagugcagguag | 22 |
| | bovine | caaagugcuuacagugcagguagu | 24 |
| | rat | caaagugcuuacagugcagguag | 65 |
| miR-92a | human | uauugcacuugucccggccugu | 28 |
| | bovine | uauugcacuugucccggccugu | 34 |
| | rat | uauugcacuugucccggccug | 66 |
| miR-146a | human | ugagaacugaauuccauggguu | 53 |
| | bovine | ugagaacugaauuccauagguugu | 58 |
| | rat | ugagaacugaauuccauggguu | 67 |
| miR-16 | human | uagcagcacguaaauauuggcg | 68 |
| | rat | uagcagcacguaaauauuggcg | 69 |
| miR-16a | bovine | uagcagcacguaaauauuggug | 70 |
| miR-18a | human | uaaggugcaucuagugcagauag | 71 |
| | bovine | uaaggugcaucuagugcagaua | 72 |
| | rat | uaaggugcaucuagugcagauag | 73 |
| miR-19b | human | ugugcaaauccaugcaaaacuga | 74 |
| | bovine | ugugcaaauccaugcaaaacuga | 75 |
| | rat | ugugcaaauccaugcaaaacuga | 76 |
| miR-20a | human | uaaagugcuuauagugcagguag | 77 |
| | bovine | uaaagugcuuauagugcagguag | 78 |
| | rat | uaaagugcuuauagugcagguag | 79 |
| miR-21 | human | uagcuuaucagacugauguuga | 80 |
| | bovine | uagcuuaucagacugauguugacu | 81 |
| | rat | uagcuuaucagacugauguuga | 82 |
| miR-23a | human | aucacauugccagggauuucc | 83 |
| | bovine | aucacauugccagggauuucca | 84 |
| | rat | aucacauugccagggauuucc | 85 |
| miR-27a | human | uucacaguggcuaaguuccgc | 86 |
| | rat | uucacaguggcuaaguuccgc | 87 |
| miR-27a-3p | bovine | uucacaguggcuaaguuccg | 88 |
| miR-27a-5p | bovine | agggcuuagcugcuugugagca | 89 |
| miR-27b | human | uucacaguggcuaaguucugc | 90 |
| | bovine | uucacaguggcuaaguucugc | 91 |
| | rat | uucacaguggcuaaguucugc | 92 |
| miR-29a | human | uagcaccaucugaaaucgguua | 93 |
| | bovine | cuagcaccaucugaaaucgguua | 94 |
| | rat | uagcaccaucugaaaucgguua | 95 |

**Table 2**

| miRNA | Human or animal | Sequence | SEQ ID NO |
|---|---|---|---|
| miR-29b | human | uagcaccauuugaaaucaguguu | 96 |
| | bovine | uagcaccauuugaaaucaguguu | 97 |
| | rat | uagcaccauuugaaaucaguguu | 98 |
| miR-29c | human | uagcaccauuugaaaucgguua | 99 |
| | bovine | uagcaccauuugaaaucgguua | 100 |
| | rat | uagcaccauuugaaaucgguua | 101 |
| miR-29c* | human | ugaccgauuucuccugguguuc | 102 |
| | rat | ugaccgauuucuccugguguuc | 103 |
| miR-30a | human | uguaaacauccucgacuggaag | 104 |
| | bovine | uguaaacauccucgacuggaagcu | 105 |
| | rat | uguaaacauccucgacuggaag | 106 |
| miR-30c | human | uguaaacauccuacacucucagc | 107 |
| | bovine | uguaaacauccuacacucucagc | 108 |
| | rat | uguaaacauccuacacucucagc | 109 |
| miR-30d | human | uguaaacauccccgacuggaag | 110 |
| | bovine | uguaaacauccccgacuggaagcu | 111 |
| | rat | uguaaacauccccgacuggaag | 112 |
| miR-30e* | human | cuuucagucggauguuuacagc | 113 |
| | rat | cuuucagucggauguuuacagc | 114 |
| miR-33a | human | gugcauuguaguugcauugca | 115 |
| | bovine | gugcauuguaguugcauugca | 116 |
| miR-33 | rat | gugcauuguaguugcauugca | 117 |
| miR-34b | human | caaucacuaacuccacugccau | 118 |
| | bovine | aggcaguguaauuagcugauug | 119 |
| | rat | uaggcaguguaauuagcugauug | 120 |
| miR-93 | human | caaagugcuguucgugcagguag | 121 |
| | bovine | caaagugcuguucgugcaggua | 122 |
| | rat | caaagugcuguucgugcagguag | 123 |
| miR-100 | human | aacccguagauccgaacuugug | 124 |
| | bovine | aacccguagauccgaacuugug | 125 |
| | rat | aacccguagauccgaacuugug | 126 |
| miR-101 | human | uacaguacugugauaacugaa | 127 |
| miR-101a | bovine | uacaguacugugauaacugaa | 128 |
| | rat | uacaguacugugauaacugaa | 129 |
| miR-101b | rat | uacaguacugugauagcugaa | 130 |
| miR-106b | bovine | uaaagugcugacagugcagau | 131 |
| | rat | uaaagugcugacagugcagau | 132 |
| miR-130b | human | cagugcaaugaugaaagggcau | 133 |
| | bovine | cagugcaaugaugaaagggcau | 134 |
| | rat | cagugcaaugaugaaagggcau | 135 |
| miR-140-3p | human | uaccacaggguagaaccacgg | 136 |
| miR-140* | rat | uaccacaggguagaaccacgg | 137 |
| miR-141 | human | uaacacugucugguaaagaugg | 138 |
| | bovine | uaacacugucugguaaagaugg | 139 |
| | rat | uaacacugucugguaaagaugg | 140 |
| miR-143 | human | ugagaugaagcacuguagcuc | 141 |
| | bovine | ugagaugaagcacuguagcucg | 142 |
| | rat | ugagaugaagcacuguagcuca | 143 |

**Table 3**

| miRNA | Human or animal | Sequence | SEQ ID NO |
|---|---|---|---|
| miR-185 | human | uggagagaaaggcaguuccuga | 144 |
| | bovine | uggagagaaaggcaguuccuga | 145 |
| | rat | uggagagaaaggcaguuccuga | 146 |
| miR-186 | human | caaagaauucuccuuuugggcu | 147 |
| | bovine | caaagaauucuccuuuugggcu | 148 |
| | rat | caaagaauucuccuuuugggcu | 149 |
| miR-192 | human | cugaccuaugaauugacagcc | 150 |
| | bovine | cugaccuaugaauugacagccag | 151 |
| | rat | cugaccuaugaauugacagcc | 152 |
| miR-193a-3p | human | aacuggccuacaaagucccagu | 153 |
| | bovine | aacuggccuacaaagucccagu | 154 |
| miR-193 | rat | aacuggccuacaaagucccagu | 155 |
| miR-195 | human | uagcagcacagaaauauuggc | 156 |
| | bovine | uagcagcacagaaauauuggca | 157 |
| | rat | uagcagcacagaaauauuggc | 158 |
| miR-200a | human | uaacacugucugguaacgaugu | 159 |
| | bovine | uaacacugucugguaacgauguu | 160 |
| | rat | uaacacugucugguaacgaugu | 161 |
| miR-205 | human | uccuucauuccaccggagucug | 162 |
| | bovine | uccuucauuccaccggagucug | 163 |
| | rat | uccuucauuccaccggagucug | 164 |
| miR-218 | human | uugugcuugaucuaaccaugu | 165 |
| | rat | uugugcuugaucuaaccaugu | 166 |
| miR-219-5p | human | ugauuguccaaacgcaauucu | 167 |
| | rat | ugauuguccaaacgcaauucu | 168 |
| miR-221 | human | agcuacauugucugcuggguuuc | 169 |
| | bovine | agcuacauugucugcuggguuu | 170 |
| | rat | agcuacauugucugcuggguuuc | 171 |
| miR-301a | human | cagugcaauaguauugucaaagc | 172 |
| | bovine | cagugcaauaguauugucaaagcau | 173 |
| | rat | cagugcaauaguauugucaaagc | 174 |
| miR-322 | rat | cagcagcaauucauguuuugga | 175 |
| miR-340 | human | uuauaaagcaaugagacugauu | 176 |
| | bovine | uccgucucaguuacuuuauagcc | 177 |
| miR-340-5p | rat | uuauaaagcaaugagacugauu | 178 |
| miR-361 | human | uuaucagaaucuccagggguac | 179 |
| | bovine | uuaucagaaucuccagggguac | 180 |
| | rat | uuaucagaaucuccagggguac | 181 |
| miR-429 | human | uaauacugucugguaaaaccgu | 182 |
| | bovine | uaauacugucugguaaugccgu | 183 |
| | rat | uaauacugucugguaaugccgu | 184 |
| miR-455 | human | uaugugccuuuggacuacaucg | 185 |
| | bovine | uaugugccuuuggacuacauc | 186 |
| | rat | uaugugccuuuggacuacaucg | 187 |
| miR-466b | rat | uauguguguguguauguccaug | 188 |
| miR-497 | human | cagcagcacacugugguuugu | 189 |
| | bovine | cagcagcacacugugguuugua | 190 |
| | rat | cagcagcacacugugguuugua | 191 |

**Table 4**

| miRNA | Human or animal | Sequence | SEQ ID NO |
|---|---|---|---|
| miR-500 | human | uaauccuugcuaccugggugaga | 192 |
| | bovine | uaauccuugcuaccugggugaga | 193 |
| | rat | aaugcaccugggcaaggguuca | 194 |
| miR-503 | human | uagcagcgggaacaguucugcag | 195 |
| | rat | uagcagcgggaacaguacugcag | 196 |
| miR-532 | bovine | caugccuugaguguaggaccgu | 198 |
| miR-532-5p | human | caugccuugaguguaggaccgu | 197 |
| | rat | caugccuugaguguaggacugu | 199 |
| miR-542-3p | human | ugugacagauugauaacugaaa | 200 |
| | rat | ugugacagauugauaacugaaa | 201 |
| I et-7a | human | ugagguaguagguuguauaguu | 202 |
| | bovine | ugagguaguagguuguauaguu | 203 |
| | rat | ugagguaguagguuguauaguu | 204 |
| I et-7a* | human | cuauacaaucuacugucuuuc | 205 |
| | bovine | cuauacaaucuacugucuuuc | 206 |
| | rat | ugagguaguagguuguauaguu | 207 |
| I et-7b | human | ugagguaguagguugugugguu | 208 |
| | bovine | ugagguaguagguugugugguu | 209 |
| | rat | ugagguaguagguugugugguu | 210 |
| I et-7c | human | ugagguaguagguuguaugguu | 211 |
| | bovine | ugagguaguagguuguaugguu | 212 |
| | rat | ugagguaguagguuguaugguu | 213 |
| I et-7d | human | agagguaguagguugcauaguu | 214 |
| | bovine | agagguaguagguugcauaguu | 215 |
| | rat | agagguaguagguugcauaguu | 216 |
| I et-7e | human | ugagguaggagguuguauaguu | 217 |
| | bovine | ugagguaggagguuguauagu | 218 |
| | rat | ugagguaggagguuguauaguu | 219 |
| let-7f | human | ugagguaguagauuguauaguu | 220 |
| | bovine | ugagguaguagauuguauaguu | 221 |
| | rat | ugagguaguagauuguauaguu | 222 |
| let-7i | human | ugagguaguaguuugugcuguu | 38 |
| | bovine | ugagguaguaguuugugcuguu | 40 |
| | rat | ugagguaguaguuugugcuguu | 223 |
| miR-10a | human | uacccuguagauccgaauuugug | 224 |
| | bovine | uacccuguagauccgaauuugug | 225 |
| miR-10a-5p | rat | uacccuguagauccgaauuugug | 226 |
| miR-10b | human | uacccuguagaaccgaauuugug | 227 |
| | bovine | uacccuguagaaccgaauuugug | 228 |
| | rat | cccuguagaaccgaauuugugu | 229 |
| miR-15b | human | uagcagcacaucaugguuuaca | 230 |
| | bovine | uagcagcacaucaugguuuaca | 231 |
| | rat | uagcagcacaucaugguuuaca | 232 |
| miR-19a | human | ugugcaaaucuaugcaaaacuga | 233 |
| | bovine | ugugcaaaucuaugcaaaacuga | 234 |
| | rat | ugugcaaaucuaugcaaaacuga | 235 |
| miR-20a* | human | acugcauuaugagcacuuaaag | 236 |
| | rat | acugcauuacgagcacuuaca | 237 |
| miR-22 | human | aagcugccaguugaagaacugu | 238 |

**Table 5**

| miRNA | Human or animal | Sequence | SEQ ID NO |
|---|---|---|---|
| miR-22-3p | bovine | aagcugccaguugaagaacug | 239 |
| miR-22 | rat | aagcugccaguugaagaacugu | 240 |
| miR-23b | human | aucacauugccagggauuacc | 241 |
| | rat | aucacauugccagggauuacc | 242 |
| miR-23b-5p | bovine | ggguuccuggcaugcugauuu | 243 |
| miR-23b-3p | bovine | aucacauugccagggauuaccac | 244 |
| miR-24 | human | uggcucaguucagcaggaacag | 245 |
| | bovine | gugccuacugagcugauaucagu | 246 |
| | rat | uggcucaguucagcaggaacag | 247 |
| miR-25 | human | cauugcacuugucucggucuga | 248 |
| | bovine | cauugcacuugucucggucuga | 249 |
| | rat | cauugcacuugucucggucuga | 250 |
| miR-26a | human | uucaaguaauccaggauaggcu | 251 |
| | bovine | uucaaguaauccaggauaggcu | 252 |
| | rat | uucaaguaauccaggauaggcu | 253 |
| miR-26b | human | uucaaguaauucaggauaggu | 254 |
| | bovine | uucaaguaauucaggauagguu | 472 |
| | rat | uucaaguaauucaggauaggu | 255 |
| miR-28 | human | aaggagcucacagucuauugag | 256 |
| | bovine | aaggagcucacagucuauugag | 257 |
| | rat | aaggagcucacagucuauugag | 258 |
| miR-30a* | human | cuuucagucggauguuugcagc | 259 |
| | rat | cuuucagucggauguuugcagc | 260 |
| miR-30b | human | uguaaacauccuacacucagcu | 261 |
| miR-30b-5p | bovine | uguaaacauccuacacucagcu | 262 |
| | rat | uguaaacauccuacacucagcu | 263 |
| miR-30c-1* | human | cugggagaggguuguuuacucc | 264 |
| | rat | cugggagaggguuguuuacucc | 265 |
| miR-30c-2* | human | cugggagaaggcuguuuacucu | 266 |
| | rat | cugggagaaggcuguuuacucu | 267 |
| miR-30e | human | uguaaacauccuugacuggaag | 268 |
| | rat | uguaaacauccuugacuggaag | 270 |
| miR-30e-5p | bovine | uguaaacauccuugacuggaagcu | 269 |
| miR-31 | human | aggcaagaugcuggcauagcu | 271 |
| | bovine | aggcaagaugcuggcauagcu | 272 |
| | rat | aggcaagaugcuggcauagcug | 273 |
| miR-34a | human | uggcagugucuuagcugguugu | 274 |
| | bovine | uggcagugucuuagcugguugu | 275 |
| | rat | uggcagugucuuagcugguugu | 276 |
| miR-96 | human | uuuggcacuagcacauuuuugcu | 277 |
| | bovine | uuuggcacuagcacauuuuugcu | 278 |
| | rat | uuuggcacuagcacauuuuugcu | 279 |
| miR-98 | human | ugagguaguaaguuguauuguu | 280 |
| | bovine | ugagguaguaaguuguauuguu | 281 |
| | rat | ugagguaguaaguuguauuguu | 282 |
| miR-99a | human | aacccguagauccgaucuugug | 283 |
| | bovine | aacccguagauccgaucuugu | 284 |
| | rat | aacccguagauccgaucuugug | 285 |

**Table 6**

| miRNA | Human or animal | Sequence | SEQ ID NO |
|---|---|---|---|
| miR-99b | human | cacccguagaaccgaccuugcg | 286 |
| | bovine | cacccguagaaccgaccuugcg | 287 |
| | rat | cacccguagaaccgaccuugcg | 288 |
| miR-103 | human | agcagcauuguacagggcuauga | 289 |
| | bovine | agcagcauuguacagggcuauga | 290 |
| | rat | agcagcauuguacagggcuauga | 291 |
| miR-107 | human | agcagcauuguacagggcuauca | 292 |
| | bovine | agcagcauuguacagggcuauc | 293 |
| | rat | agcagcauuguacagggcuauca | 294 |
| miR-125a-3p | human | acaggugagguucuugggagcc | 43 |
| | rat | acaggugagguucuugggagcc | 295 |
| miR-125a-5p | human | ucccugagacccuuuaaccuguga | 42 |
| | rat | ucccugagacccuuuaaccuguga | 296 |
| miR-125a | bovine | ucccugagacccuuuaaccugug | 48 |
| miR-125b | human | ucccugagacccuaacuuguga | 46 |
| | bovine | ucccugagacccuaacuuguga | 51 |
| miR-125b-5p | rat | ucccugagacccuaacuuguga | 297 |
| miR-125b-1* | human | acggguuaggcucuugggagcu | 298 |
| miR-125b-3p | rat | acggguuaggcucuugggagcu | 299 |
| miR-128 | human | ucacagugaaccggucucuuu | 300 |
| | bovine | ucacagugaaccggucucuuu | 301 |
| | rat | ucacagugaaccggucucuuu | 302 |
| miR-130a | human | cagugcaauguuaaaagggcau | 303 |
| | bovine | cagugcaauguuaaaagggcau | 304 |
| | rat | cagugcaauguuaaaagggcau | 305 |
| miR-133a | human | uuugguccccuucaaccagcug | 306 |
| | bovine | uuugguccccuucaaccagcug | 307 |
| | rat | uuugguccccuucaaccagcug | 308 |
| miR-133b | human | uuugguccccuucaaccagcua | 309 |
| | bovine | uuugguccccuucaaccagcua | 310 |
| | rat | uuugguccccuucaaccagcua | 311 |
| miR-134 | human | ugugacugguugaccagagggg | 312 |
| | bovine | ugugacugguugaccagagugg | 313 |
| | rat | ugugacugguugaccagagggg | 314 |
| miR-139-3p | human | ggagacgcggcccuguuggagu | 315 |
| | rat | uggagacgcggcccuguuggag | 316 |
| miR-140 | human | cagugguuuuacccuaugguag | 317 |
| | bovine | uaccacaggguagaaccacgga | 318 |
| | rat | cagugguuuuacccuaugguag | 319 |
| miR-146b | human | ugagaacugaauuccauaggcu | 55 |
| | bovine | ugagaacugaauuccauaggcugu | 60 |
| | rat | ugagaacugaauuccauaggcugu | 320 |
| miR-148b | human | ucagugcaucacagaacuuugu | 321 |
| | bovine | ucagugcaucacagaacuuugu | 322 |
| miR-148b-3p | rat | ucagugcaucacagaacuuugu | 323 |
| miR-151 | human | ucgaggagcucacagucuagu | 324 |
| | bovine | cuagacugaagcuccuugagg | 325 |
| | rat | cuagacugaagcuccuugagg | 326 |

**Table 7**

| miRNA | Human or animal | Sequence | SEQ ID NO |
|---|---|---|---|
| miR-152 | human | ucagugcaugacagaacuugg | 327 |
| | bovine | ucagugcaugacagaacuuggg | 328 |
| | rat | ucagugcaugacagaacuugg | 329 |
| miR-181a | human | aacauucaacgcugucggugagu | 7 |
| | bovine | aacauucaacgcugucggugaguu | 13 |
| | rat | aacauucaacgcugucggugagu | 330 |
| miR-181a* | human | accaucgaccguugauuguacc | 331 |
| | rat | accaucgaccguugauuguacc | 332 |
| miR-181b | human | aacauucauugcugucggugggu | 10 |
| | bovine | aacauucauugcugucgguggguu | 16 |
| | rat | aacauucauugcugucggugggu | 333 |
| miR-181c | human | aacauucaaccugucggugagu | 334 |
| | bovine | aacauucaaccugucggugaguuu | 335 |
| | rat | aacauucaaccugucggugagu | 336 |
| miR-181d | human | aacauucauuguugucggugggu | 337 |
| | bovine | aacauucauuguugucggugggu | 338 |
| | rat | aacauucauuguugucggugggu | 339 |
| miR-182 | human | uuuggcaaugguagaacucacacu | 340 |
| | bovine | uuuggcaaugguagaacucacacu | 341 |
| | rat | uuuggcaaugguagaacucacaccg | 342 |
| miR-183 | human | uauggcacugguagaauucacu | 343 |
| | bovine | uauggcacugguagaauucacug | 344 |
| | rat | uauggcacugguagaauucacu | 345 |
| miR-188 | human | caucccuugcaugguggaggg | 346 |
| | bovine | caucccuugcaugguggagggu | 347 |
| | rat | caucccuugcaugguggaggg | 348 |
| miR-196c | rat | uagguaguuucguguuguuggg | 349 |
| miR-199a-3p | human | acaguagucugcacauugguua | 350 |
| | bovine | acaguagucugcacauugguua | 351 |
| | rat | acaguagucugcacauugguua | 352 |
| miR-200b | human | uaauacugccugguaaugauga | 353 |
| | bovine | uaauacugccugguaaugaug | 354 |
| | rat | uaauacugccugguaaugaugac | 355 |
| miR-200c | human | uaauacugccggguaaugaugga | 356 |
| | bovine | uaauacugccggguaaugaugga | 357 |
| | rat | uaauacugccggguaaugaugg | 358 |
| miR-203 | human | gugaaauguuuaggaccacuag | 359 |
| | rat | gugaaauguuuaggaccacuag | 360 |
| miR-204 | human | uucccuuugucauccuaugccu | 361 |
| | bovine | uucccuuugucauccuaugccu | 362 |
| | rat | uucccuuugucauccuaugccu | 363 |
| miR-206 | human | uggaauguaaggaagugugugg | 364 |
| | bovine | uggaauguaaggaagugugugg | 365 |
| | rat | uggaauguaaggaagugugugg | 366 |
| miR-210 | human | cugugcgugugacagcggcuga | 367 |
| | bovine | acugugcgugugacagcggcuga | 368 |
| | rat | cugugcgugugacagcggcuga | 369 |

**Table 8**

| miRNA | Human or animal | Sequence | SEQ ID NO |
|---|---|---|---|
| miR-212 | human | uaacagucuccagucacggcc | 370 |
| | bovine | accuuggcucuagacugcuuacu | 371 |
| | rat | uaacagucuccagucacggcca | 372 |
| miR-214 | human | acagcaggcacagacaggcagu | 373 |
| | bovine | acagcaggcacagacaggcagu | 374 |
| | rat | acagcaggcacagacaggcag | 375 |
| miR-222 | human | agcuacaucuggcuacugggu | 376 |
| | bovine | agcuacaucuggcuacugggu | 377 |
| | rat | agcuacaucuggcuacugggu | 378 |
| miR-223 | human | ugucaguuugucaaauacccca | 18 |
| | bovine | ugucaguuugucaaauacccca | 20 |
| | rat | ugucaguuugucaaauacccc | 379 |
| miR-290 | rat | cucaaacuaugggggcacuuuuu | 380 |
| miR-291a-5p | rat | caucaaaguggaggcccucucu | 381 |
| miR-292-5p | rat | acucaaacugggggcucuuuug | 382 |
| miR-294 | rat | cucaaaauggaggcccuaucu | 383 |
| miR-296-5p | human | agggcccccccucaauccugu | 384 |
| miR-296* | rat | agggcccccccucaauccugu | 385 |
| miR-320a | human | aaaagcuggguugagagggcga | 386 |
| miR-320 | bovine | aaaagcuggguugagagggcga | 387 |
| | rat | aaaagcuggguugagagggcga | 388 |
| miR-324-3p | human | acugccccaggugcugcugg | 389 |
| | rat | ccacugccccaggugcugcugg | 390 |
| miR-324 | bovine | cgcauccccuagggcauuggugu | 392 |
| miR-324-5p | human | cgcauccccuagggcauuggugu | 391 |
| | rat | cgcauccccuagggcauuggugu | 393 |
| miR-327 | rat | ccuugaggggcaugagggu | 394 |
| miR-328 | human | cuggcccucucugcccuuccgu | 395 |
| | bovine | cuggcccucucugcccuuccgu | 396 |
| | rat | cuggcccucucugcccuuccgu | 397 |
| miR-331 | human | gccccugggccuauccuagaa | 398 |
| | bovine | gccccugggccuauccuagaa | 399 |
| | rat | gccccugggccuauccuagaa | 400 |
| miR-340-3p | rat | uccgucucaguuacuuuauagcc | 403 |
| miR-341 | rat | ucggucgaucggucggucggu | 404 |
| miR-342 | bovine | ucucacacagaaaucgcacccaucu | 406 |
| miR-342-3p | human | ucucacacagaaaucgcacccgu | 405 |
| | rat | ucucacacagaaaucgcacccgu | 407 |
| miR-345 | human | gcugacuccuaguccagggcuc | 408 |
| miR-345-5p | bovine | gcugacuccuaguccagugcu | 409 |
| | rat | ugcugaccccuaguccagugc | 410 |
| miR-347 | rat | ugucccucugggucgccca | 411 |
| miR-352 | rat | agaguaguagguugcauagua | 412 |
| miR-365 | human | uaaugccccuaaaaauccuuau | 413 |
| | rat | uaaugccccuaaaaauccuuau | 415 |
| miR-365-3p | bovine | uaaugccccuaaaaauccuuau | 414 |

**Table 9**

| miRNA | Human or animal | Sequence | SEQ ID NO |
|---|---|---|---|
| miR-370 | human | gccugcugggguggaaccuggu | 416 |
| | bovine | gccugcugggguggaaccuggu | 417 |
| | rat | gccugcugggguggaaccugguu | 418 |
| miR-375 | human | uuuguucguucggcucgcguga | 419 |
| | bovine | uuuuguucguucggcucgcguga | 420 |
| | rat | uuuguucguucggcucgcguga | 421 |
| miR-378 | human | acuggacuuggagucagaagg | 422 |
| | bovine | acuggacuuggagucagaaggc | 423 |
| | rat | acuggacuuggagucagaagg | 424 |
| miR-378* | human | cuccugacuccagguccugugu | 425 |
| | rat | cuccugacuccagguccugugu | 426 |
| miR-425 | human | aaugacacgaucacucccguuga | 427 |
| | bovine | augacacgaucacucccguuga | 428 |
| | rat | aaugacacgaucacucccguuga | 429 |
| miR-465 | rat | uauuuagaacggugcuggugug | 430 |
| miR-483 | human | ucacuccucuccucccgucuu | 431 |
| | bovine | ucacuccucuccucccgucuu | 432 |
| | rat | ucacuccuccccucccgucuugu | 433 |
| miR-484 | human | ucaggcucaguccccucccgau | 434 |
| | bovine | ucaggcucaguccccucccgau | 435 |
| | rat | ucaggcucaguccccucccgau | 436 |
| miR-494 | human | ugaaacauacacgggaaaccuc | 437 |
| | bovine | ugaaacauacacgggaaaccuc | 438 |
| | rat | ugaaacauacacgggaaaccu | 439 |
| miR-542-5p | human | ucggggaucaucaugucacgaga | 440 |
| | bovine | ucggggaucaucaugucacgag | 441 |
| | rat | cucggggaucaucaugucacga | 442 |
| miR-652 | human | aauggcgccacuaggguugug | 443 |
| | rat | aauggcgccacuaggguugug | 444 |
| miR-672 | human | ugagguugguguacuguguguga | 445 |
| | rat | ugagguugguguacuguguguga | 446 |
| miR-685 | bovine | ucaauggcugaggugagguac | 447 |
| | rat | ucaauggcugaggugaggcac | 448 |
| miR-760 | human | cggcucugggucugugggga | 449 |
| | bovine | ccccucaguccaccagagcccg | 450 |
| miR-760-3p | rat | cggcucugggucugugggga | 451 |
| miR-872 | human | aagguuacuuguuaguucagg | 452 |
| | rat | aagguuacuuguuaguucagg | 453 |
| miR-874 | human | cugcccuggcccgagggaccga | 454 |
| | bovine | cugcccuggcccgagggaccga | 455 |
| | rat | cugcccuggcccgagggaccga | 456 |
| miR-1224-5p | human | gugaggacucgggaggugg | 457 |
| miR-1224 | bovine | gugaggacucgggagguggag | 458 |
| | rat | gugaggacuggggagguggag | 459 |
| miR-193* | rat | ugggucuuugcgggcaagauga | 460 |
| miR-193a-5p | human | ugggucuuugcgggcgagauga | 461 |
| | bovine | ugggucuuugcgggcgagauga | 462 |
| miR-409-3p | human | gaauguugcucggugaaccccu | 463 |
| | rat | aauguugcucggugaacccc | 464 |

**Table 10**

| miRNA | Human or animal | Sequence | SEQ ID NO |
|---|---|---|---|
| miR-409 | bovine | agguuacccgagcaacuuugcau | 465 |
| miR-664 | human | uauucauuuauccccagccuaca | 466 |
| | bovine | caggcugggguguguguggaug | 467 |
| | rat | uauucauuuacuccccagccua | 468 |
| miR-877 | human | guagaggagauggcgcaggg | 469 |
| | bovine | guagaggagauggcgcaggg | 470 |
| | rat | guagaggagauggcgcaggg | 471 |
| miR-15a | human | uagcagcacauaaugguuugug | 473 |
| | bovine | uagcagcacauaaugguuugu | 474 |
| miR-16b | bovine | uagcagcacguaaauauuggc | 475 |
| miR-30f | bovine | uguaaacacccuacacucucagcu | 476 |
| miR-106 | bovine | aaaagugcuuacagugcaggua | 477 |
| miR-126 | human | ucguaccgugaguaauaaugcg | 478 |
| | bovine | cguaccgugaguaauaaugcg | 479 |
| | rat | ucguaccgugaguaauaaugcg | 480 |
| miR-129-3p | human | aagcccuuaccccaaaaagcau | 481 |
| | bovine | aagcccuuaccccaaaaagcau | 482 |
| miR-184 | human | uggacggagaacugauaagggu | 483 |
| | bovine | uggacggagaacugauaagggu | 484 |
| | rat | uggacggagaacugauaagggu | 485 |
| miR-196a | human | uagguaguuucauguuguuggg | 486 |
| | bovine | uagguaguuucauguuguuggg | 487 |
| | rat | uagguaguuucauguuguuggg | 488 |
| miR-338 | human | uccagcaucagugauuuuguug | 489 |
| | bovine | uccagcaucagugauuuuguuga | 490 |
| | rat | uccagcaucagugauuuuguuga | 491 |
| miR-362-5p | human | aauccuuggaaccuaggugugagu | 492 |
| | bovine | aauccuuggaaccuaggugugagu | 493 |
| miR-362 | rat | aauccuuggaaccuaggugugaau | 494 |
| miR-452 | human | aacuguuugcagaggaaacuga | 495 |
| | bovine | uguuugcagaggaaacugagac | 496 |
| miR-486 | human | uccuguacugagcugccccgag | 497 |
| | bovine | uccuguacugagcugccccgag | 498 |
| miR-584 | human | uuaugguuugccugggacugag | 499 |
| | bovine | ugguuugccugggacugag | 500 |
| miR-708 | human | aaggagcuuacaaucuagcuggg | 501 |
| | bovine | aaggagcuuacaaucuagcuggg | 502 |
| | rat | aaggagcuuacaaucuagcuggg | 503 |
| miR-1300b | bovine | ucgagaaggaggcugcug | 504 |
| miR-1307 | human | acucggcguggcgucggucgug | 401 |
| | bovine | acucggcguggcgucggucgug | 402 |

The miRNA is not limited to those having the aforementioned sequences, the miRNA may include substitutions, deletions, insertions, additions or inversions of one or several nucleotides, so long as the miRNA has the function as the miRNA, i.e., the miRNA can regulate expression of target genes. Specifically, examples of such a miRNA include RNAs having a nucleotide sequence showing a homology of 80% or more, preferably 90% or more, more preferably 95% or more, to any of the aforementioned sequences.

The amount of miRNA may be an absolute amount or a relative amount. The relative amount may be a relative amount based on an average amount in animals, or may be a relative amount observed after ingestion of a diet based on the amount observed before the ingestion. For the measurement of the amount of nucleic acid, methods usually used for measurement of miRNA amount such as quantitative reverse transcription PCR (qRT-PCR) can be employed. The amount of miRNA can also be measured by the microarray method. As for extraction of miRNA from milk, methods usually used for extraction of miRNA can be employed, and a commercially available miRNA isolation kit can also be used.

Further, amount of miRNA present in milk can also be indirectly measured by measuring expression amount of the miRNA in mammary glandular cells.

Correlation of miRNA profiles in milk of a mammal and a diet ingested by the mammal or a substance contained in the diet is examined. The correlation of the miRNA profiles in milk of a mammal and a diet ingested by the mammal or a substance contained in the diet refers to correlation of the miRNA profile and presence or absense of the substance or amount of the substance. For example, if amounts of one or more kinds of miRNAs in milk of an animal which has ingested a certain substance are larger or smaller than those observed in the animal which has not ingested the substance, the substance and the miRNA profiles have positive or negative correlation, respectively. Further, if ingestion of a certain substance does not affect miRNA profiles, the substance and the miRNA profiles do not correlate with eath other.

Specifically, for example, when miRNA profiles in milk observed before and after ingestion of a diet are compared, amount or amounts of one kind, preferably two kinds or more, more preferably five kinds or more, of miRNAs observed after the ingestion are larger than those observed before the ingestion, it is judged that the diet increases amounts of miRNAs existing in milk.

In another embodiment, miRNA profiles in milk and miRNA profiles in serum or plasma are compared, and if amount of miRNA contained in both of milk and serum or plasma is increased by ingestion of the diet at a higher degree in milk as compared to that observed in serum or plasma, it is judged that the diet increases amount of the miRNA present in milk. The degree of increase in amount of miRNA in milk is, for example, 1.2 times or more, preferably 2 times or more, more preferably 5 times or more, still more preferably 10 times or more, of that observed in serum or plasma.

The diet may consist of a single substance or may be a composition, so long as it can be orally ingested.

Further, "before ingestion" and "after ingestion" may mean "before and after one time of ingestion of diet", or "before and after two or more times of ingestion of diet".

Further, two or more times of ingestion of diet may be two or more times of ingestion of the same diet, or ingestion of two or more kinds of diets.

The diet may be ingested according to a planned scheme or freely ingested. In the latter case, correlation of the diet and miRNA profiles in milk can be examined by hearing content of ingested diet in the case of human. When the diet is ingested or administered according to a planned scheme, the diet can be considered as a "test sample". The diet may be a usual diet or a usual diet containing a test substance. Amount of diet to be ingested, time of ingestion, and number of times of ingestion are not particularly limited.

If a diet that increases amount of miRNA in milk is chosen, a substance that is contained in the diet and increases amount of the miRNA in milk can be identified in the same manner as that mentioned above. Further, if a diet that decreases amount of miRNA in milk is chosen, a substance that is contained in the diet and decreases amount of the miRNA in milk can be identified in the same manner as that mentioned above.

If a diet or a substance that increases or decreases amount of miRNA in milk is identified, a diet that increases or decreases amount of the miRNA in milk can be designed. That is, it is thought that a diet that increases amount of miRNA in milk or a substance contained therein is preferred for production of milk having an immunostimulating action.

Screening for a diet or a substance providing production of breast milk having an immunoregulatory action, or a diet or a substance unsuitable for production of breast milk having an immunoregulatory action can be performed as described above. As shown in the examples described later, presence of various kinds of miRNAs was confirmed in colostrum of rat and bovine. This supports the concept of the present invention that it is expected that oral administration of miRNA provides an immunoregulatory action. Further, as shown in the examples described later, when *Bifidobacterium* bacteria (*Bifidobacterium longum*) were orally administered to rats, amounts of 52 kinds of miRNAs increased.

It is known that *Bifidobacterium* bacteria function as probiotics, and have, in particular, an immunoregulatory action. Therefore, the fact that the administration of the *Bifidobacterium* bacteria increased amounts of miRNAs in milk also supports the involvement of miRNAs in milk in immunoregulation. Demonstration of increase in amounts of miRNAs in milk induced by administration of the *Bifidobacterium* bacteria, i.e., correlation of the *Bifidobacterium* bacteria and miRNA profiles, shows that the screening method of the present invention is feasible. Further, although there were also miRNAs of which amounts in milk were not changed by administration of the *Bifidobacterium* bacteria, a possibility that amounts of those miRNAs may be increased by another kind of diet or a substance contained therein is not denied.

As probiotic functions of *Bifidobacterium* bacteria, there are known prophylaxis or amelioration of respiratory tract infection, acute infectious diarrhea, antibiotic-associated diarrhea, *Clostridium dificile-*associated diarrhea, necrotising enterocolitis, traveler's diarrhea, *Helicobacter pylori* infection, and so forth (The Journal of Nutrition, 2010 Mar;140(3):698S-712S. Epub 2010 Jan 27). It is suggested that miRNA of which amount in milk is increased by administration of *Bifidobacterium* bacteria not only regulates immunity, but also exhibits functions similar to the aforementioned probiotic functions in animals that ingested them.

By giving a diet or a substance that increases amount of miRNA in milk chosen as described above to a mammal, and collecting milk from the animal, milk having an immunostimulating action or milk of which immunostimulating action is enhanced can be obtained. Further, by reducing or avoiding ingestion by a mammal of a diet or a substance that decreases amount of miRNA in milk chosen as described above, an immunostimulating action of milk can be enhanced, or decrease of an immunostimulating action can be prevented.

Further, ingestion of a diet or a substance that increases amount of miRNA in milk and reduction or avoidance of ingestion of a diet or a substance that decreases amount of miRNA in milk may be combined. Further, by giving a diet or a substance that decreases amount of miRNA in milk chosen as described above to a mammal, and collecting milk from the animal, milk having an immunosuppressive action or milk of which immunostimulating action is decreased can be obtained. Further, by reducing or avoiding ingestion by a mammal of a diet or a substance that increases amount of miRNA in milk chosen as described above, an immunosuppressive action of milk can be enhanced, or an immunostimulating action of milk can be decreased. Further, ingestion of a diet or a substance that decreases amount of miRNA in milk and reduction or avoidance of ingestion of a diet or a substance that increases amount of miRNA in milk may be combined.

By processing milk having an immunoregulatory action obtained as described above, dairy products having an immunoregulatory action can be produced.

Type of the dairy products is not particularly limited, so long as miRNAs can exist in it with maintaining the functions thereof, and examples include processed milk, infant formula, milk beverages, powdered infant formula, fermented milk, cream, butter, cheese, ice cream, and so forth. As the dairy product, a dairy product for infants or little children is preferred.

According to the present invention, there was demonstrated presence in milk of miRNAs, especially miRNAs which have been known to participate in enhancement of immunity, such as development of immunity, antiallergy, anti-inflammation, and defense against infection. In addition, it is well known that breast milk gives an immunostimulating action to an infant who ingested it. Therefore, it is rationally predicted that the miRNA participating in immunoregulation can regulate immunity of organism such as human who ingested it. Since miRNA is a substance that regulates expression of various genes, it is considered that transfer of such regulatory molecules from a mother to an infant is extremely significant for, in particular, infants having an underdeveloped immune system.

A composition for oral ingestion having an immunostimulating action, which may be prepared by adding miRNA to a base for composition for oral ingestion.

Examples of miRNA include miR-10, miR-15, miR-16, miR-17, miR-18, miR-19, miR-20, miR-21, miR-22, miR-23, miR-24, miR-25, miR-26, miR-27, miR-28, miR-29, miR-30, miR-31, miR-33, miR-34, miR-92, miR-93, miR-96, miR-98, miR-99, miR-100, miR-101, miR-103, miR-106, miR-107, miR-125, miR-126, miR-128, miR-129, miR-130, miR-133, miR-134, miR-139, miR-140, miR-141, miR-143, miR-146, miR-148, miR-151, miR-152, miR-155, miR-181, miR-182, miR-183, miR-184, miR-185, miR-186, miR-188, miR-192, miR-193, miR-195, miR-196, miR-199, miR-200, miR-203, miR-204, miR-205, miR-206, miR-210, miR-212, miR-214, miR-218, miR-219, miR-221, miR-222, miR-223, miR-290, miR-291, miR-292, miR-294, miR-296, miR-301, miR-320, miR-322, miR-324, miR-327, miR-328, miR-331, miR-338, miR-340, miR-341, miR-342, miR-345, miR-347, miR-352, miR-361, miR-362, miR-365, miR-370, miR-375, miR-378, miR-409, miR-425, miR-429, miR-452, miR-455, miR-465, miR-466, miR-483, miR-484, miR-486, miR-494, miR-497, miR-500, miR-503, miR-532, miR-542, miR-584, miR-652, miR-664, miR-672, miR-685, miR-708, miR-760, miR-872, miR-874, miR-877, miR-1224, miR-1300, miR-1307, let-7a, let-7b, let-7c, let-7d, let-7e, let-7f, let-7i, and so forth.

Among the aforementioned miRNAs, miR-15, miR-16, miR-17, miR-18, miR-19, miR-20, miR-21, miR-23, miR-24, miR-26, miR-27, miR-29, miR-30, miR-33, miR-34, miR-92, miR-93, miR-99, miR-100, miR-101, miR-106, miR-107, miR-125, miR-130, miR-140, miR-141, miR-143, miR-146, miR-155, miR-181, miR-185, miR-186, miR-192, miR-193, miR-195, miR-200, miR-205, miR-210, miR-218, miR-219, miR-221, miR-222, miR-223, miR-301, miR-322, miR-340, miR-361, miR-370, miR-429, miR-455, miR-466, miR-497, miR-500, miR-503, miR-532, miR-542, let-7d, and let-7i are preferred, and miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d, and let-7i are more preferred.

The miRNA may consist of a single kind of miRNA or arbitrary two or more kinds of miRNAs.

The base for composition for oral ingestion is not particularly limited so long as an orally ingestible or administrable base in which miRNA can exist with maintaining functions thereof is chosen, and examples include foodstuffs, drinks, drug bases, animal feeds, and so forth.

Foodstuffs may be in any form, and include drinks. Foodstuffs include foodstuffs for adults, foodstuffs for infants, foodstuffs for little children, and so forth.

Examples of the foodstuffs for adults include enteral nutrients, fluid diets such as concentrated fluid diets, nutritional supplementary foods, and so forth.

Examples of the foodstuffs for infants or the foodstuffs for little children include, for example, modified milks (for example, infant formula, infant formula for low birth weight infants, follow-up formula, etc. as well as infant formula for allergic infants, non-lactose milk, special milk for inborn errors of metabolism infants, etc., and dried milk prepared from these), powders for supplement of breast milk or powdered infant formula, baby food, and so forth.

The infant formula referred to here are foodstuffs produced by using milk or dairy products as main raw materials, and adding nutrients required for infants, and are mainly used as alternative food for breast milk in infancy, and as alternative food for breast milk or nutritional complementary food in childhood. Other examples thereof include foodstuffs produced for the purpose of contributing to nutritional ingestion suitable for infants with a specific inherent or acquired disease.

miRNA is relatively resistant to freeze-thaw, low pH such as acidic conditiond of pH 1, and RNases such as RNase A and RNase T, and thus is suitable as an active ingredient to be added to foodstuffs. The stability at a low pH suggests that miRNA molecules are resistant to the infant's intragastric environment, and can be absorbed by the intestinal tract, which is one of the main immune organs of infants, and thus they can affect the immune system of infants. Further, storage and freeze-thaw of breast milk do not denature miRNA, and this is nutritionally important for low birth weight infants and hospitalized infants, who are usually given cryopreserved breast milk. Furthermore, the resistance of miRNA to RNases suggests that miRNA may exist in a complex such as exosome and microvesicle in breast milk.

From the aforementioned findings, it sounds that mothers give to infants such custom-made breast milk that the infants can adapt to the environment. There is a report suggesting that breast milk-derived exosomes increase the number of Foxp3+ CD4+ CD25+ regulatory T cells. If immunity-related miRNAs are contained in breast milk exosomes, they may possibly contribute to the increase in Foxp3+ CD4+ CD25+ regulatory T cells in the alimentary canal of infants. This is because the immunity-related miRNAs detected in breast milk such as miR-181a and miR-181b are highly expressed, and they are involved in T cell differentiation. Furthermore, since it is known that miR-181 and miR-155 abundantly contained in breast milk induce B cell differentiation, and there is almost no miR-150, which suppresses B cell differentiation, in breast milk, miRNAs in breast milk may induce differentiation of B cells.

Although content of miRNA in the composition is not particularly limited, and may be appropriately chosen, it is, for example, 10 to 10,000 ng/ml, preferably 20 to 10,000 ng/ml, more preferably 50 to 10,000 ng/ml, in total. Further, amount of miRNA to be ingested is, for example, 5 µg to 120 mg/day, preferably 10 µg to 120 mg/day, more preferably 25 µg to 120 mg/day, in total.

miRNA can be obtained by preparing a partially double-stranded RNA as a precursor of miRNA (pri-miRNA), and digesting it with a Dicer enzyme. As the Dicer enzyme, commercially available enzymes can be used. The double-stranded RNA can be prepared by, for example, a RNA polymerase reaction using a double-stranded DNA having a complementary sequence as a template. The double-stranded DNA can be prepared by amplification based on PCR using a chromosomal DNA of mammal as a template and primers designed so as to be able to amplify the sequence of miRNA.

miRNA can be obtained by digesting the double-stranded RNA obtained as described above with a Dicer enzyme or the like.

Further, miRNA can also be prepared by chemical synthesis. That is, miRNA can be obtained by synthesizing a sense strand and an antisense strand and annealing them.

Further, a double-stranded RNA that allows generation of a target miRNA by means of an endogenous Dicer enzyme of mammal may be added to the composition for oral ingestion.

When the composition for oral ingestion is a pharmaceutical agent, the composition can be prepared by combining a miRNA with pharmaceutically acceptable carriers for oral
administration. The form of the pharmaceutical preparation is not particularly limited, and examples include tablet, pill, powder, solution, suspension, emulsion, granule, capsule, syrup, and so forth. For the formulation, additives widely used for usual pharmaceutical agents as pharmaceutical carriers for oral administration such as excipients, binders, disintegrating agents, lubricants, stabilizers, corrigents, diluents, and surfactants can be used. Further, unless the effect of the present invention is degraded, miRNA may be used together with another drug having an immunoregulatory action.

Although amount of miRNA contained in the pharmaceutical agent is not particularly limited, it is, for example, 2 µg/kg to 2 mg/kg, preferably 4 µg/kg to 2 mg/kg, more preferably 10 µg/kg to 2 mg/kg, in total.

When the composition for oral ingestion is a foodstuff, it may be for any of various uses utilizing an immunostimulating action. Examples of the use include, for example, uses as foodstuffs suitable for persons showing reduced resistance, uses as foodstuffs or drinks useful for reduction and elimination of risk factors of various diseases caused by immune depression, and so forth.

The foodstuffs or drinks can be marketed as foodstuffs attached with an indication describing that the foodstuffs are used for immunoregulation.

The aforementioned term "indication" includes all actions for informing consumers the aforementioned use, and any indications reminding or analogizing the aforementioned use fall within the scope of the "indication" of the present invention regardless of purpose, content, objective article, medium etc. of the indication. However, the indication is preferably made with an expression that allows consumers to directly recognize the aforementioned use. Specific examples include actions of indicating the aforementioned use on goods or packages of goods relating to the foodstuff of the present invention, actions of assigning, delivering, displaying for the purpose of assigning or delivering or importing such goods or packages of goods on which the aforementioned use is indicated, displaying or distributing advertisements, price lists or business papers relating the goods, or providing information including those as contents with indicating the aforementioned use by an electromagnetic method (Internet etc.) and so forth.

The indication is preferably an indication approved by the administration etc. (for example, an indication in a form based on an approval, which is qualified on the basis of any of various legal systems provided by the administration), and it is particularly preferably an indication on advertisement materials at the sales spots such as packages, containers, catalogs, pamphlets and POPs, others documents and so forth.

Examples of the indication further include, for example, indications as health food, functional food, enteric nutritive food, food for special dietary uses, food with nutrient function claims, quasi-drug and so forth as well as indications approved by the Ministry of Health, Labor and Welfare, for example, indications approved on the basis of the system of food for specified health uses and similar systems. Examples of the latter include indications as food for specified health uses, indications as food for specified health uses with qualified health claims, indications of influence on body structures and functions, indications of reduction of disease risk claims and so forth, and more precisely, typical examples include indications as food for specified health uses (especially indications of use for health) provided in the enforcement regulations of Health Promotion Law (Japan Ministry of Health, Labor and Welfare, Ministerial ordinance No. 86, April 30, 2003) and similar indications.

### Examples

Hereafter, the present invention will be further specifically explained with reference to examples.

### Example 1: Analysis of miRNAs in breast milk

Human breast milk was centrifuged at 2,000 x g for 10 minutes to remove cells and large precipitates, and the supernatant except for the lipids constituting a surface layer was further centrifuged at 12,000 x g for 30 minutes to remove cell debris and small dusts. Total RNA was extracted from the supernatant using the mirVana miRNA isolation kit according to the manufacturer's protocol. Extraction of RNAs from serum was performed in the same manner as that used for the breast milk.

The extracted RNAs were analyzed by using a bioanalyzer. Although a considerable amount of RNAs were contained in breast milk, ribosomal RNAs (18S rRNA, 28S rRNA) were scarsely contained, or were not contained at all.

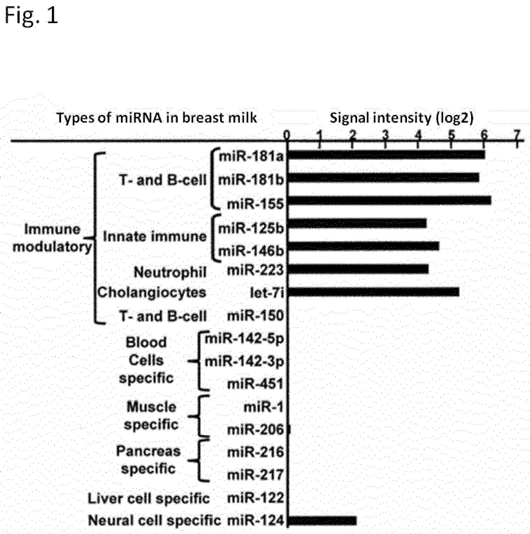

miRNAs were detected by using a microarray analysis system (one produced by Agilent Technologies was used). Expression level of miRNAs was analyzed by using GeneSpring GX11.0 (produced by Agilent Technologies). The results are shown in Fig. 1. As a result, miR-181a, miR-181b, miR-155, miR-125b, miR-146b, miR-223, and let-7i were detected in marked level. miR-150, which controls T cells and B cells, could not be detected. Further, a plurality of organ-specific miRNAs such as miR-122 (liver), miR-216, miR-217 (pancreas), miR-142-5p, and miR-142-3p (hematopoietic cell) could hardly be detected. Furthermore, miR-124 (brain) was detected in a small amount.

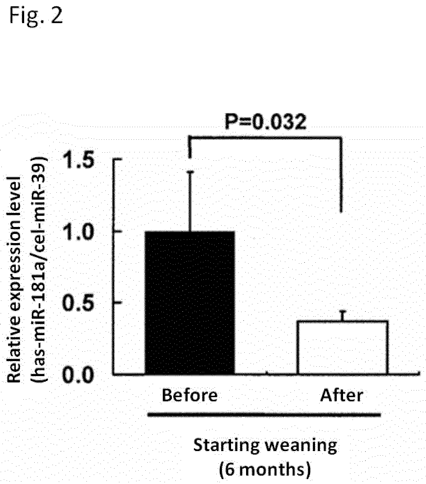

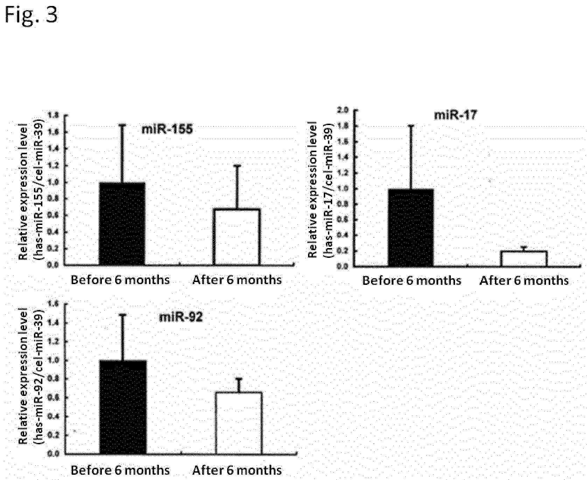

The results of comparison of miR-181a levels analyzed by quatitative RT-PCR in breast milk for first six months after the birth (n = 5) and next six months (n = 13) are shown as Fig. 2. The results of similar analyses conducted for miR-155, miR-17 and miR-92a are also shown in Fig. 3. In order to normalize the variations among the samples induced by the RNA isolation process, denatured cel-miR-39 (synthesized by Qiagen), which is a synthesized miRNA of a nematode (*Caenorhabditis elegans*), was added to the samples (at an oligonucleotide amount of 25 fmol in the total volume of 5 ml), and the amounts of miRNAs are shown as relative amounts based on the cel-miR-39 amount (the same shall apply to the following experiments).

As a result, the amount of miR-181a was larger in the milk of the first six months after the birth as compared to that in the milk of the six months thereafter (Fig. 2). Similar tendencies were also observed for miR-155, miR-17, and miR-92a (Fig. 3).

As the primers for RT-PCR, those produced by Applied Biosystems and identified by the following Assay IDs were used.
- miR-181a: 000480
- miR-155: 002623
- miR-17: 002308
- miR-92a: 000431
- Cel-miR-39: 000200

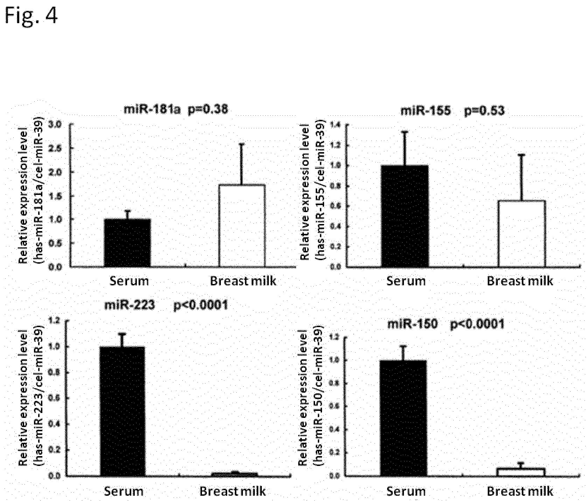

The results of comparison of immunity-related miRNA levels in breast milk and serum of seven healthy humans within 6 months post-partum are shown in Fig. 4 (breast milk: n = 5, serum: n = 6). The miRNA profiles in the breast milk are different from those in the serum. For example, miR-223, which is miRNA that controls granulocytes, existed at the highest level in normal human serum and plasma, whereas the expression amount thereof in the breast milk was extremely very lower as compared to that in the serum. Further, miR-146b which does not abundantly exist in the serum abundantly existed in the breast milk.

On the other hand, miR-181 and miR-155 abundantly existed in the breast milk at expression amounts comparable to those observed in the serum. It is interesting that a plurality of kinds of immunity-related miRNAs was highly expressed in the breast milk of post-partum six months, which is a stage before ingestion of baby food.

Intercellular transfer of miRNAs indicates that not only miRNAs control intracellular molecules, but also they are molecules playing a role in communication between cells like cytokines. The aforementioned results suggest that miRNAs are "genetic materials" that can be transferred from a mother to a child. It is calculated that about 0.15 pg/L/day (1.3 x 10⁷ copies/L/day) of miR-181 is ingested by an infant via breast milk.

In addition, it was found that miRNA profiles in breast milks of different mothers were similar, as a result of a cluster analysis.

### Example 2: Physicochemical properties of miRNA

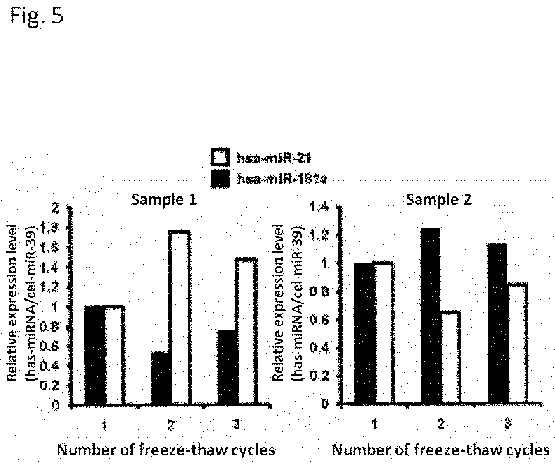

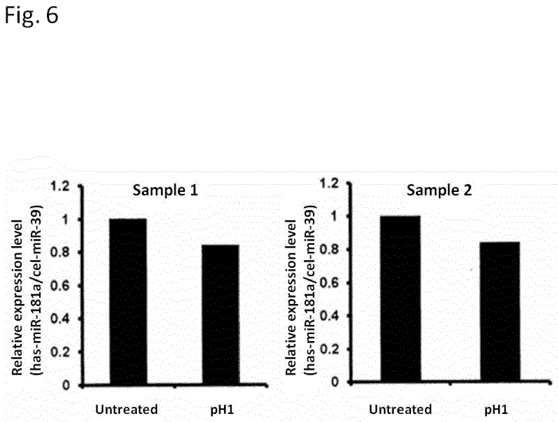

Breast milk was left standing at room temperature for 24 hours, or repeatedly subjected to freezing (-20°C) and thawing up to 3 times. The levels of miRNAs (miR-21, miR-181a) were measured by TaqMan qRT-PCR. The results are shown in Fig. 5. Further, breast milk was treated in a low pH solution (pH 1) for 3 hours, and the miRNA level (miR-181a) was measured by TaqMan qRT-PCR before and after the treatment. The results are shown in Fig. 6.

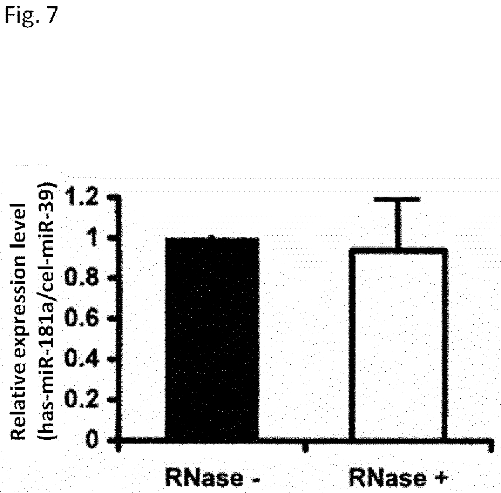

Further, to breast milk, an RNase A/T solution (mixed solution of RNase A (500 U/ml) and RNase T1 (20,000 U/ml), produced by Ambion) was added in a volume of 2% of the breast milk, the mixture was treated at 37°C for 3 hours, and the miRNA level (miR-181a) was measured by TaqMan qRT-PCR before and after the treatment. The results are shown in Fig. 7.

As the primers for TaqMan qRT-PCR, those produced by Applied Biosystems and identified by the following Assay IDs were used.
- miR-181a: 000480
- miR-21: 000397
- Cel-miR-39: 000200

It was demonstrated that miRNAs were relatively stable to freeze-thaw, low pH, and RNases.

### Example 3: Identification of diet or substance providing production of milk having immunoregulatory action

SD rats at pregnancy day 9 to 10 were purchased, and a suspension of a *Bifidobacterium* bacteria, *Bifidobacterium longum* BB536 (ATCC BAA-999) in PBS (phosphate buffered saline) (1 x 10⁹ cfu/ml) was orally administered to the rats in a test group (n = 3) everyday in a volume of 1 ml/day per rat in the period of pregnancy days 13 to 20.

Further, as a control group (n = 3), PBS was administered everyday in a volume of 1 ml per rat. The *B*. *longum* ATCC BAA-999 strain can be purchased from American Type Culture Collection (Address: 12301 Parklawn Drive, Rockville, Maryland 20852, United States of America).

All the rats gave birth on pregrancy day 21, and they were milked under anesthesia with ether on the first day after the birth. The obtained colostrum sample was centrifuged twice at 1,200 x g and 4°C for 10 minutes to remove the lipid layer and cell debris.

Then, the supernatant was centrifuged at 21,500 x g and 4°C for 40 minutes, and further centrifuged for 1 hour under the same conditions to remove the casein fraction and thereby obtain milk serum. Total RNA was obtained from the obtained milk serum sample by using miRNeasy Mini Kit (produced by Qiagen).

By using 100 ng of the obtained RNA sample, miRNAs were detected in a conventional manner using a microarray analysis system (produced by Agilent Technologies). The results were analyzed by using GeneSpring GX11.0 (produced by Agilent Technologies).

When statistical analysis of the microarray data was conducted by using GeneSpring GX11.0, it was found that the number of types of the microRNAs of which expression was confirmed in the test group and the control group in which they were detected was 155 in total. Such microRNAs are as follows. In addition, miR-150 was not detected.

MicroRNAs of which expression was confirmed in the test group and the control group, 155 types:
miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, emir-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p, miR-542-3p
let-7a, let-7a*, let-7b, let-7c, let-7d, let-7e, let-7f, let-7i, miR-10 (miR-10a-5p, miR-10b), miR-15 (miR-15b), miR-19 (miR-19a), miR-20 (miR-20a*), miR-22, miR-23 (miR-23b), miR-24, miR-25, miR-26 (miR-26a, miR-26b), miR-28, miR-30 (miR-30a*, miR-30b-5p, miR-30c-1*, miR-30c-2*, miR-30e), miR-31, miR-34 (miR-34a), miR-96, miR-98, miR-99 (miR-99a, miR-99b), miR-103, miR-107, miR-125 (miR-125a-3p, miR-125a-5p, miR-125b-3p, miR-125b-5p), miR-128, miR-130 (miR-130a), miR-133 (miR-133a, miR-133b), miR-134, miR-139 (miR-139-3p), miR-140, miR-146 (miR-146b), miR-148 (miR-148b-3p), miR-151, miR-152, miR-181 (miR-181a, miR-181a*, miR-181b, miR-181c, miR-181d), miR-182, miR-183, miR-188, miR-196 (miR-196c), miR-199 (miR-199a-3p), miR-200 (miR-200b, miR-200c), miR-203, miR-204, miR-206, miR-210, miR-212, miR-214, miR-222, miR-223, miR-290, miR-291 (miR-291a-5p), miR-292 (miR-292-5p), miR-294, miR-296 (miR-296*), miR-320, miR-324 (miR-324-3p, miR-324-5p), miR-327, miR-328, miR-331, miR-340 (miR-340-3p), miR-341, miR-342 (miR-342-3p), miR-345 (miR-345-5p), miR-347, miR-352, miR-365, miR-370, miR-375, miR-378 (miR-378, miR-378*), miR-425, miR-465, miR-483, miR-484, miR-494, miR-542 (miR-542-5p), miR-652, miR-672, miR-685, miR-760 (miR-760-3p), miR-872, miR-874, miR-1224

The miRNAs listed with parenthesized indications following the miR-No. have subtypes, and subtypes indicated in the parentheses actually expressed.

Further, when expression amounts of the aforementioned microRNAs in the *Bifidobacterium* bacteria BB 536-administered group and the control group were statistically compared by using the Mann-Whitney U-test, it was found that the following 52 types of microRNAs increased in the *Bifidobacterium* bacteria BB 536-administered group at a probability level of less than 5%. Magnitudes of variation in expression of the miRNAs are shown in Table 11.

MicroRNAs of which increase was confirmed in the *Bifidobacterium* bacteria BB 536-administered group, 52 types:
miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p, miR-542-3p.

**Table 11**

| | Mann-Whitney U test | | | |
|---|---|---|---|---|
| | Systematic name | p-Value | Regulation | Magnitude of variation |
| 1 | rno-miR-16 | 0.049535 | up | 1.67 |
| 2 | rno-miR-17-5p | 0.049535 | up | 1.83 |
| 3 | rno-miR-18a | 0.049535 | up | 2.03 |
| 4 | rno-miR-19b | 0.049535 | up | 1.64 |
| 5 | rno-miR-20a | 0.049535 | up | 2.04 |
| 6 | rno-miR-21 | 0.049535 | up | 1.92 |
| 7 | rno-miR-23a | 0.049535 | up | 1.68 |
| 8 | rno-miR-27a | 0.049535 | up | 1.64 |
| 9 | rno-miR-27b | 0.049535 | up | 1.98 |
| 10 | rno-miR-29a | 0.049535 | up | 1.53 |
| 11 | rno-miR-29b | 0.049535 | up | 1.92 |
| 12 | rno-miR-29c | 0.049535 | up | 1.64 |
| 13 | rno-miR-29c* | 0.049535 | up | 1.72 |
| 14 | rno-miR-30a | 0.049535 | up | 1.70 |
| 15 | rno-miR-30c | 0.049535 | up | 1.94 |
| 16 | rno-miR-30d | 0.049535 | up | 1.50 |
| 17 | rno-miR-30e* | 0.049535 | up | 2.01 |
| 18 | rno-miR-33 | 0.036904 | up | 2.53 |
| 19 | rno-miR-34b | 0.049535 | up | 3.02 |
| 20 | rno-miR-92a | 0.049535 | up | 2.09 |
| 21 | rno-miR-93 | 0.049535 | up | 1.70 |
| 22 | rno-miR-100 | 0.049535 | up | 2.08 |
| 23 | rno-miR-101a | 0.049535 | up | 2.81 |
| 24 | rno-miR-101b | 0.049535 | up | 1.97 |
| 25 | rno-miR-106b | 0.049535 | up | 1.74 |
| 26 | rno-miR-130b | 0.046302 | up | 4.83 |
| 27 | rno-miR-140* | 0.049535 | up | 1.83 |
| 28 | rno-miR-141 | 0.049535 | up | 1.76 |
| 29 | rno-miR-143 | 0.049535 | up | 2.16 |
| 30 | rno-miR-146a | 0.049535 | up | 1.95 |
| 31 | rno-miR-185 | 0.049535 | up | 1.74 |
| 32 | rno-miR-186 | 0.049535 | up | 1.70 |
| 33 | rno-miR-192 | 0.049535 | up | 2.37 |
| 34 | rno-miR-193 | 0.049535 | up | 2.10 |
| 35 | rno-miR-195 | 0.049535 | up | 2.37 |
| 36 | rno-miR-200a | 0.049535 | up | 1.88 |
| 37 | rno-miR-205 | 0.049535 | up | 1.47 |
| 38 | rno-miR-218 | 0.049535 | up | 1.91 |
| 39 | rno-miR-219-5p | 0.049535 | up | 1.73 |
| 40 | rno-miR-221 | 0.049535 | up | 2.02 |
| 41 | rno-miR-301a | 0.049535 | up | 1.59 |
| 42 | rno-miR-322 | 0.049535 | up | 1.72 |
| 43 | rno-miR-340-5p | 0.049535 | up | 3.12 |
| 44 | rno-miR-361 | 0.049535 | up | 1.83 |
| 45 | rno-miR-429 | 0.049535 | up | 1.52 |
| 46 | rno-miR-455 | 0.049535 | up | 2.33 |
| 47 | rno-miR-466b | 0.049535 | up | 1.55 |
| 48 | rno-miR-497 | 0.049535 | up | 2.41 |
| 49 | rno-miR-500 | 0.049535 | up | 1.91 |
| 50 | rno-miR-503 | 0.049535 | up | 6.91 |
| 51 | rno-miR-532-5p | 0.049535 | up | 2.78 |
| 52 | rno-miR-542-3p | 0.049535 | up | 3.13 |

As seen from the results shown in Table 11, it was found that the magnitudes of the variation observed for all the 52 types of the microRNAs of which increases were confirmed were 1.2 times or larger.

That is, it was found that the *Bifidobacterium* bacteria BB536 strain could be screened for as a diet or a substance providing production of milk having an immunoregulatory action on the basis of detection of these 52 types of microRNAs.

### Example 4: Detection of microRNAs expressed in rat colostrum

Three F344 rats on pregnancy day 14 were purchased. All the purchased rats gave birth on pregrancy day 21, and they were milked under anesthesia with ether on the second day after the birth to collect colostrum.

Each colostrum sample was centrifuged twice at 1,200 x g and 4°C for 10 minutes to remove the lipid layer and cell debris.

Then, the supernatant was centrifuged at 21,500 x g and 4°C for 40 minutes, and further centrifuged for 1 hour under the same conditions to remove the casein fraction and thereby obtain milk serum.

Total RNA was obtained from the obtained milk serum sample by using miRNeasy Mini Kit (produced by Qiagen).

The obtained RNA sample in an amount of 100 ng was used in an experiment on a microarray (produced by Agilent Technologies) in a conventional manner. The results of the microarray experiment were analyzed by using GeneSpring GX11.0 (produced by Agilent Technologies).

As a result, it was confirmed that four kinds of microRNAs (miR-193*, miR-409-3p, miR-664, miR-877) were expressed in addition to the 155 kinds of microRNAs confirmed in Example 3.

### Example 5: Detection of microRNAs expressed in bovine colostrum

Five samples of milk of Holstein cows in the period of the post-partum days 1 to 3 were prepared as colostrum samples. Further, five samples of milk of Holstein cows in the period from the post-partum day 8 to 8 months were prepared as normal milk samples.

Each of the milk samples (colostrum and normal milk) was centrifuged twice at 1,200 x g and 4°C for 10 minutes to remove the lipid layer and cell debris.

Then, the supernatant was centrifuged at 21,500 x g and 4°C for 40 minutes, and further centrifuged for 1 hour under the same conditions to remove the casein fraction and thereby obtain milk serum.

Total RNA was obtained from the obtained milk serum sample by using miRNeasy Mini Kit (produced by Qiagen).

The obtained RNA sample in an amount of 20 ng was used in an experiment on a microarray (produced by Agilent Technologies) in a conventional manner. The results of the microarray experiment were analyzed by using GeneSpring GX11.0 (produced by Agilent Technologies).

As a result, expression of 102 kinds in total of miRNAs was confirmed in the colostrum samples and the normal milk samples. In particular, among the 102 kinds of miRNAs, expression of 49 kinds of miRNAs was confirmed only in the colostrum.

The 49 kinds of microRNAs of which expression was confirmed only in the colostrum samples are mentioned below.

MicroRNAs of which expression was confirmed only in the colostrums, 49 types:
let-7d, let-7i, miR-15a, miR-15b, miR-16b, miR-17-3p, miR-19b, miR-21, miR-23b-3p, miR-24-3p, miR-26b, miR-27b, miR-30a-5p, miR-30c, miR-30f, miR-34a, miR-99a, miR-106, miR-106b, miR-107, miR-125b, miR-126, miR-129-3p, miR-130a, miR-130b, miR-140, miR-155, miR-181b, miR-184, miR-193a-3p, miR-193a-5p, miR-196a, miR-210, miR-222, miR-223, miR-338, miR-361, miR-362-5p, miR-370, miR-429, miR-452, miR-486, miR-500, miR-532, miR-584, miR-708, miR-877, miR-1300b, miR-1307

### Industrial Applicability

According to the present invention, a diet or a substance contained therein providing production of milk having an immunoregulatory action can be screened for. The present invention also provides a method for producing dairy products having an immunoregulatory action.
## Claims

1. A method for screening for a diet or a substance providing production of breast milk having an immunostimulating action, which comprises:
comparing microRNA profiles in milk of rat observed before and after ingestion of a diet or a substance, and when amount of one or more kinds of microRNA in the milk observed after the ingestion of the diet or the substance is higher than that observed before the ingestion, judging that the diet or the substance increases the amount of the microRNA in the milk,
wherein the microRNA is selected from the group consisting of miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p and miR-542-3p.

2. A method for screening for a diet or a substance providing production of breast milk having an immunostimulating action, which comprises:
comparing microRNA profiles in milk of bovine observed before and after ingestion of a diet or a substance, and when amount of one or more kinds of microRNA in the milk observed after the ingestion of the diet or the substance is higher than that observed before the ingestion, judging that the diet or the substance increases the amount of the microRNA in the milk,
wherein the microRNA is selected from the group consisting of miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d and let-7i.

3. The method according to claim 1, which further comprises:
comparing microRNA profiles in the milk of the rat observed before and after ingestion of the diet or the substance with microRNA profiles in serum or plasma of the rat observed before and after ingestion of the diet or the substance, respectively, and
when the amount of microRNA contained in both the milk and the serum or plasma is increased by ingestion of the diet or substance, judging that the diet or the substance increases the amount of the microRNA in the milk.

4. The method according to claim 2, which further comprises:
comparing microRNA profiles in the milk of the bovine observed before and after ingestion of the diet or the substance with microRNA profiles in serum or plasma of the bovine observed before and after ingestion of the diet or the substance, respectively, and
when the amount of microRNA contained in both the milk and the serum or plasma is increased by ingestion of the diet or substance, judging that the diet or the substance increases the amount of the microRNA in the milk.

5. A method for producing milk or dairy products having an immunostimulating action, which comprises:
the step of giving *Bifidobacterium* bacteria to bovine, and
the step of collecting milk from the bovine, wherein the collected milk has, compared to untreated animals, an increased amount of microRNA selected from the group consisting of miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d and let-7i.

6. A method for producing milk or dairy products having an immunostimulating action, which comprises:
the step of giving *Bifidobacterium* bacteria to rat, and
the step of collecting milk from the rat, wherein the collected milk has, compared to untreated animals, an increased amount of microRNA selected from the group consisting of miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p and miR-542-3p.

## Patentansprüche

1. Verfahren zum Screenen auf eine Kost oder einen Stoff, die/der die Herstellung von Muttermilch ermöglicht, die eine immunstimulierende Wirkung aufweist, wobei das Verfahren umfasst:
Vergleichen von microRNA-Profilen in Rattenmilch, die vor und nach der Aufnahme einer Kost oder eines Stoffs ermittelt wurden, und Beurteilen, dass die Kost oder der Stoff die Menge an microRNA in der Milch erhöht, wenn die Menge einer oder mehrerer Arten von microRNA, die nach der Aufnahme der Kost oder des Stoffs in der Milch ermittelt wurde, höher ist als die, die vor der Aufnahme ermittelt wurde,
wobei die microRNA ausgewählt ist aus der Gruppe bestehend aus miR-16, miR-17-5p, miR-18 (miR-18a), miR-19 (miR-19b), miR-20 (miR-20a), miR-21, miR-23 (miR-23a), miR-27 (miR-27a, miR27-b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p und miR-542-3p.

2. Verfahren zum Screenen auf eine Kost oder einen Stoff, die/der die Herstellung von Muttermilch ermöglicht, die eine immunstimulierende Wirkung aufweist, wobei das Verfahren umfasst:
Vergleichen von microRNA-Profilen in Rindermilch, die vor und nach der Aufnahme einer Kost oder eines Stoffs ermittelt wurden, und Beurteilen, dass eine Kost oder ein Stoff die Menge an microRNA in der Milch erhöht, wenn die Menge einer oder mehrerer Arten von microRNA, die nach der Aufnahme der Kost oder des Stoffs in der Milch ermittelt wurde, höher ist als die, die vor der Aufnahme ermittelt wurden, wobei die microRNA ausgewählt ist aus der Gruppe bestehend aus miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d und let-7i.

3. Verfahren nach Anspruch 1, das des Weiteren umfasst:
Vergleichen von microRNA-Profilen in der Rattenmilch, die vor und nach der Aufnahme der Kost oder des Stoffs ermittelt wurden, mit microRNA-Profilen, die vor und nach der Aufnahme der Kost bzw. des Stoffs im Serum oder Plasma der Ratte ermittelt wurden, und
Beurteilen, dass die Kost oder der Stoff die Menge an microRNA in der Milch erhöht, wenn die Menge an microRNA, die sowohl in der Milch als auch dem Serum oder Plasma enthalten ist, durch die Aufnahme der Kost oder des Stoffs erhöht ist.

4. Verfahren nach Anspruch 2, das des Weiteren umfasst:
Vergleichen von microRNA-Profilen in der Kuhmilch, die vor und nach der Aufnahme der Kost oder des Stoffs ermittelt wurden, mit microRNA-Profilen, die vor und nach der Aufnahme der Kost oder des Stoffs im Serum oder Plasma des Rinds ermittelt wurden, und
Beurteilen, dass die Kost oder der Stoff die Menge an microRNA in der Milch erhöht, wenn die Menge an microRNA, die sowohl in der Milch als auch dem Serum oder Plasma enthalten ist, durch die Aufnahme der Kost oder des Stoffs erhöht ist.

5. Verfahren zur Herstellung von Milch oder Molkereiprodukten, die eine immunstimulierende Wirkung aufweist/aufweisen, wobei das Verfahren umfasst:
den Schritt des Verabreichens von *Bifidobacterium-*Bakterien an ein Rind, und
den Schritt des Gewinnens von Milch von dem Rind, wobei die gewonnene Milch, im Vergleich zu unbehandelten Tieren, eine erhöhte Menge an microRNA aufweist, die ausgewählt ist aus der Gruppe bestehend aus miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d und let-7i.

6. Verfahren zur Herstellung von Milch oder Molkereiprodukten, die eine immunstimulierende Wirkung aufweist/aufweisen, wobei das Verfahren umfasst:
den Schritt des Verabreichens von *Bifidobacterium-*Bakterien an eine Ratte und
den Schritt des Gewinnens von Milch von der Ratte, wobei die gewonnene Milch, im Vergleich zu unbehandelten Tieren, eine erhöhte Menge an microRNA aufweist, die ausgewählt ist aus der Gruppe bestehend aus miR-16, miR-17-5p, miR-18 (miR-18a), miR-19 (miR-19b), miR-20 (miR-20a), miR-21, miR-23 (miR-23a), miR-27 (miR-27a, miR27-b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p und miR-542-3p.

## Revendications

1. Procédé de criblage d'une alimentation ou d'une substance permettant la production de lait maternel ayant une action immunostimulante comprenant des étapes consistant à :
comparer des profils de micro ARN dans du lait de rat observés avant et après l'ingestion d'une alimentation ou d'une substance, et, lorsque la quantité d'un ou de plusieurs types de micro ARN dans le lait observée après l'ingestion de l'alimentation ou de la substance est supérieure à celle observée avant l'ingestion, considérer que l'alimentation ou la substance augmente la quantité de micro ARN dans le lait,
le micro ARN étant choisi dans le groupe constitué par les micro ARN suivants :
miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p et miR-542-3p.

2. Procédé de criblage d'une alimentation ou d'une substance permettant la production de lait maternel ayant une action immunostimulante comprenant des étapes consistant à :
comparer les profils de micro ARN dans du lait de bovin observés avant et après l'ingestion d'une alimentation ou d'une substance, et lorsque la quantité d'un ou de plusieurs types de micro ARN dans le lait observée après l'ingestion de l'alimentation ou de la substance est supérieure à celle observée avant l'ingestion, considérer que l'alimentation ou la substance augmente la quantité de micro ARN dans le lait,
le micro ARN étant choisi dans le groupe formé par les micro ARN suivants :
miR-15, miR-16, miR-19, miR-21, miR-23, miR-24, miR-26, miR-27, miR-30, miR-34, miR-99, miR-106, miR-107, miR-12 5, miR-130, miR-140, miR-181, miR-193, miR-210, miR-222, miR-223, miR-361, miR-370, miR-429, miR-500, miR-532, let-7d et let-7i.

3. Procédé conforme à la revendication 1,
comprenant en outre des étapes consistant à :
comparer respectivement les profils de micro ARN dans le lait du rat observés avant et après l'ingestion de l'alimentation ou de la substance avec des profils de micro ARN dans le sérum ou le plasma du rat observés avant et après l'ingestion de l'alimentation ou de la substance, et
lorsque la quantité de micro ARN contenue dans le lait et dans le sérum ou le plasma est augmentée par ingestion de l'alimentation ou de la substance, considérer que l'alimentation ou la substance augmente la quantité de micro ARN dans le lait.

4. Procédé conforme à la revendication 2,
comprenant en outre des étapes consistant à :
comparer respectivement des profils de micro ARN dans le lait du bovin observés avant et après l'ingestion de l'alimentation ou de la substance avec des profils de micro ARN dans le sérum ou le plasma du bovin observés avant et après l'ingestion de l'alimentation ou de la substance, et lorsque la quantité de micro ARN renfermée dans le lait et le sérum ou le plasma est augmenté par l'ingestion de l'alimentation ou de la substance, considérer que l'alimentation ou la substance augmente la quantité de micro ARN dans le lait.

5. Procédé d'obtention de lait ou de produits laitiers ayant une action immunostimulante comprenant :
une étape consistant à administrer des bactéries *bifidobactérium* à des bovins, et
une étape consistant à recueillir le lait des bovins, le lait recueilli ayant, par comparaison avec des animaux non traités, une quantité augmentée de micro ARN choisis dans le groupe formé par les micro ARN suivants :
miR-15, miR-16, miR-19, miR-21, miR-23, miR-24, miR-26, miR-27, miR-30, miR-34, miR-99, miR-106, miR-107, miR-125, miR-130, miR-140, miR-181, miR-193, miR-210, miR-222, miR-223, miR-361, miR-370, miR-429, miR-500, miR-532, let-7d et let-7i.

6. Procédé d'obtention de lait ou de produits laitiers ayant une action immunostimulante comprenant :
une étape consistant à administrer des bactéries *bifidobactérium* à des rats, et
une étape consistant à recueillir du lait de ces rats, le lait recueilli ayant, par comparaison avec des animaux non traités, une quantité augmentée de micro ARN choisis dans le groupe formé par les micro ARN suivants : miR-16, miR-17-5p, miR-18 (miR-18a), miR-19 (miR-19b), miR-20 (miR-20a), miR-21, miR-23 (miR-23a), miR-27 (miR-27a, miR-27b), miR-29 (miR-29a, miR-29b, miR-29c, miR-29c*), miR-30 (miR-30a, miR-30c, miR-30d, miR-30e*), miR-33, miR-34b, miR-92a, miR-93, miR-100, miR-101 (miR-101a, miR-101b), miR-106b, miR-130b, miR-140*, miR-141, miR-143, miR-146a, miR-185, miR-186, miR-192, miR-193, miR-195, miR-200a, miR-205, miR-218, miR-219-5p, miR-221, miR-301a, miR-322, miR-340-5p, miR-361, miR-429, miR-455, miR-466b, miR-497, miR-500, miR-503, miR-532-5p et miR-542-3p.


<110> Morinaga Milk Industry Co., Ltd.
<120> Method for screening for diet providing production of milk having immunoregulatory action
<130> FP207-10120
<150> JP2009-165991
   <151> 2009-07-14
<160> 504
<170> PatentIn version 3.5
<210> 1
   <211> 65
   <212> RNA
   <213> Homo sapiens
<400> 1
   cuguuaaugc uaaucgugau agggguuuuu gccuccaacu gacuccuaca uauuagcauu    60
   aacag                                                                 65
<210> 2
   <211> 23
   <212> RNA
   <213> Homo sapiens
<400> 2
   uuaaugcuaa ucgugauagg ggu 23
<210> 3
   <211> 63
   <212> RNA
   <213> Bos taurus
<400> 3
   uguuaaugcu aaucgugaua gggguuuuua ccucggacug acuccuacau guuagcauua 60
   aca 63
<210> 4
   <211> 23
   <212> RNA
   <213> Bos taurus
<400> 4
   uuaaugcuaa ucgugauagg ggu 23
<210> 5
   <211> 110
   <212> RNA
   <213> Homo sapiens
<400> 5
   ugaguuuuga gguugcuuca gugaacauuc aacgcugucg gugaguuugg aauuaaaauc 60
   aaaaccaucg accguugauu guacccuaug gcuaaccauc aucuacucca 110
<210> 6
   <211> 110
   <212> RNA
   <213> Homo sapiens
<400> 6
   agaagggcua ucaggccagc cuucagagga cuccaaggaa cauucaacgc ugucggugag 60
   uuugggauuu gaaaaaacca cugaccguug acuguaccuu gggguccuua 110
<210> 7
   <211> 23
   <212> RNA
   <213> Homo sapiens
<400> 7
   aacauucaac gcugucggug agu 23
<210> 8
   <211> 110
   <212> RNA
   <213> Homo sapiens
<400> 8
   ccugugcaga gauuauuuuu uaaaagguca caaucaacau ucauugcugu cgguggguug     60
   aacugugugg acaagcucac ugaacaauga augcaacugu ggccccgcuu               110
<210> 9
   <211> 89
   <212> RNA
   <213> Homo sapiens
<400> 9
   cugauggcug cacucaacau ucauugcugu cgguggguuu gagucugaau caacucacug     60
   aucaaugaau gcaaacugcg gaccaaaca                                       89
<210> 10
   <211> 23
   <212> RNA
   <213> Homo sapiens
<400> 10
   aacauucauu gcugucggug ggu 23
<210> 11
   <211> 110
   <212> RNA
   <213> Bos taurus
<400> 11
   ugccagggcc aggacccagu cuucagagga cuccaaggaa cauucaacgc ugucggugag     60
   uuugggauuu gaaaaaacca ccgaccguug acuguaccuu ggguuccuua               110
<210> 12
   <211> 110
   <212> RNA
   <213> Bos taurus
<400> 12
   ugagcuccga gguugcuuca gugaacauuc aacgcugucg gugaguuugg aauuaaaaau     60
   caaaaccauc gaccguugau uguacccuau ggccaaccac caucuccacc               110
<210> 13
   <211> 24
   <212> RNA
   <213> Bos taurus
<400> 13
   aacauucaac gcugucggug aguu 24
<210> 14
   <211> 110
   <212> RNA
   <213> Bos taurus
<400> 14
   cuugggcaga gguucuuucu uaaaagguca caaucaacau ucauugcugu cgguggguug      60
   aacugugugg acaagcucac ugaacaauga gugcaacugu ggccccgcau                110
<210> 15
   <211> 89
   <212> RNA
   <213> Bos taurus
<400> 15
   cugauggcug cacucaacau ucauugcugu cgguggguuu gacuuugaau caacucacug      60
   aucaaugaau guaaacugcg gaccaaaca                                        89
<210> 16
   <211> 24
   <212> RNA
   <213> Bos taurus
<400> 16
   aacauucauu gcugucggug gguu 24
<210> 17
   <211> 110
   <212> RNA
   <213> Homo sapiens
<400> 17
   ccuggccucc ugcagugcca cgcuccgugu auuugacaag cugaguugga cacuccaugu      60
   gguagagugu caguuuguca aauaccccaa gugcggcaca ugcuuaccag                110
<210> 18
   <211> 22
   <212> RNA
   <213> Homo sapiens
<400> 18
   ugucaguuug ucaaauaccc ca 22
<210> 19
   <211> 108
   <212> RNA
   <213> Bos taurus
<400> 19
   cccagccucc ugcagugcca ugcuccgugu auuugacaag cugaguugga cacuccaugu      60
   aguaguguca guuugucaaa uaccccaagu guggcauaug ccuagcag                  108
<210> 20
   <211> 22
   <212> RNA
   <213> Bos taurus
<400> 20
   ugucaguuug ucaaauaccc ca 22
<210> 21
   <211> 84
   <212> RNA
   <213> Homo sapiens
<400> 21
   gucagaauaa ugucaaagug cuuacagugc agguagugau augugcaucu acugcaguga 60
   aggcacuugu agcauuaugg ugac 84
<210> 22
   <211> 23
   <212> RNA
   <213> Homo sapiens
<400> 22
   caaagugcuu acagugcagg uag 23
<210> 23
   <211> 84
   <212> RNA
   <213> Bos taurus
<400> 23
   gucagaauaa ugucaaagug cuuacagugc agguagugau augugcaucu acugcaguga 60
   aggcacuugu agcauuaugg ugac 84
<210> 24
   <211> 24
   <212> RNA
   <213> Bos taurus
<400> 24
   caaagugcuu acagugcagg uagu 24
<210> 25
   <211> 20
   <212> RNA
   <213> Bos taurus
<400> 25
   acugcaguga aggcacuugu 20
<210> 26
   <211> 78
   <212> RNA
   <213> Homo sapiens
<400> 26
   cuuucuacac agguugggau cgguugcaau gcuguguuuc uguaugguau ugcacuuguc     60
   ccggccuguu gaguuugg                                                   78
<210> 27
   <211> 75
   <212> RNA
   <213> Homo sapiens
<400> 27
   ucaucccugg guggggauuu guugcauuac uuguguucua uauaaaguau ugcacuuguc     60
   ccggccugug gaaga                                                      75
<210> 28
   <211> 22
   <212> RNA
   <213> Homo sapiens
<400> 28
   uauugcacuu gucccggccu gu 22
<210> 29
   <211> 96
   <212> RNA
   <213> Homo sapiens
<400> 29
   cgggccccgg gcgggcggga gggacgggac gcggugcagu guuguuuuuu cccccgccaa     60
   uauugcacuc gucccggccu ccggcccccc cggccc                               96
<210> 30
   <211> 22
   <212> RNA
   <213> Homo sapiens
<400> 30
   uauugcacuc gucccggccu cc 22
<210> 31
   <211> 68
   <212> RNA
   <213> Bos taurus
<400> 31
   ugggugggga uuuguugcac ugcuuguguu auauauaaag uauugcacuu gucccggccu     60
   guggaaga                                                              68
<210> 32
   <211> 22
   <212> RNA
   <213> Bos taurus
<400> 32
   uauugcacuu gucccggccu gu 22
<210> 33
   <211> 78
   <212> RNA
   <213> Bos taurus
<400> 33
   cuuucuacac agguugggau cgguugcaau gcuguguuuc uguaugguau ugcacuuguc    60
   ccggccuguu gaguuugg                                                  78
<210> 34
   <211> 22
   <212> RNA
   <213> Bos taurus
<400> 34
   uauugcacuu gucccggccu gu 22
<210> 35
   <211> 96
   <212> RNA
   <213> Bos taurus
<400> 35
   cgggccccgg gcgggcggga gggacgggac gcggugcagu guuguucuuu ccccugccaa    60
   uauugcacuc gucccggccu ccggcccccu cggccc                              96
<210> 36
   <211> 22
   <212> RNA
   <213> Bos taurus
<400> 36
   uauugcacuc gucccggccu cc 22
<210> 37
   <211> 84
   <212> RNA
   <213> Homo sapiens
<400> 37
   cuggcugagg uaguaguuug ugcuguuggu cggguuguga cauugcccgc uguggagaua    60
   acugcgcaag cuacugccuu gcua                                           84
<210> 38
   <211> 22
   <212> RNA
   <213> Homo sapiens
<400> 38
   ugagguagua guuugugcug uu 22
<210> 39
   <211> 84
   <212> RNA
   <213> Bos taurus
<400> 39
   cuggcugagg uaguaguuug ugcuguuggu cggguuguga cauugcccgc uguggagaua 60
   acugcgcaag cuacugccuu gcua 84
<210> 40
   <211> 22
   <212> RNA
   <213> Bos taurus
<400> 40
   ugagguagua guuugugcug uu 22
<210> 41
   <211> 86
   <212> RNA
   <213> Homo sapiens
<400> 41
   ugccagucuc uaggucccug agacccuuua accugugagg acauccaggg ucacagguga 60
   gguucuuggg agccuggcgu cuggcc 86
<210> 42
   <211> 24
   <212> RNA
   <213> Homo sapiens
<400> 42
   ucccugagac ccuuuaaccu guga 24
<210> 43
   <211> 22
   <212> RNA
   <213> Homo sapiens
<400> 43
   acaggugagg uucuugggag cc 22
<210> 44
   <211> 88
   <212> RNA
   <213> Homo sapiens
<400> 44
   ugcgcuccuc ucagucccug agacccuaac uugugauguu uaccguuuaa auccacgggu 60
   uaggcucuug ggagcugcga gucgugcu 88
<210> 45
   <211> 89
   <212> RNA
   <213> Homo sapiens
<400> 45
   accagacuuu uccuaguccc ugagacccua acuugugagg uauuuuagua acaucacaag 60
   ucaggcucuu gggaccuagg cggagggga 89
<210> 46
   <211> 22
   <212> RNA
   <213> Homo sapiens
<400> 46
   ucccugagac ccuaacuugu ga 22
<210> 47
   <211> 86
   <212> RNA
   <213> Bos taurus
<400> 47
   ugccggccuc ugcgucccug agacccuuua accugugagg acguccaggg ucacagguga 60
   gguucuuggg agccuggcgu ccggcc 86
<210> 48
   <211> 23
   <212> RNA
   <213> Bos taurus
<400> 48
   ucccugagac ccuuuaaccu gug 23
<210> 49
   <211> 88
   <212> RNA
   <213> Bos taurus
<400> 49
   cgcgcgccuc ucaaucccug agacccuaac uugugauguu uaccguuuaa auccacgggu 60
   uaggcucuug ggagcugcga gucgcgcc 88
<210> 50
   <211> 85
   <212> RNA
   <213> Bos taurus
<400> 50
   gacuuuuccu agucccugag acccuaacuu gugagguauu uuaguagcau cacaagucag      60
   gcucuuggga ccuaggcgga gggga                                            85
<210> 51
   <211> 22
   <212> RNA
   <213> Bos taurus
<400> 51
   ucccugagac ccuaacuugu ga 22
<210> 52
   <211> 99
   <212> RNA
   <213> Homo sapiens
<400> 52
   ccgaugugua uccucagcuu ugagaacuga auuccauggg uugugucagu gucagaccuc      60
   ugaaauucag uucuucagcu gggauaucuc ugucaucgu                             99
<210> 53
   <211> 22
   <212> RNA
   <213> Homo sapiens
<400> 53
   ugagaacuga auuccauggg uu 22
<210> 54
   <211> 73
   <212> RNA
   <213> Homo sapiens
<400> 54
   ccuggcacug agaacugaau uccauaggcu gugagcucua gcaaugcccu guggacucag      60
   uucuggugcc cgg                                                         73
<210> 55
   <211> 22
   <212> RNA
   <213> Homo sapiens
<400> 55
   ugagaacuga auuccauagg cu 22
<210> 56
   <211> 22
   <212> RNA
   <213> Homo sapiens
<400> 56
   ugcccugugg acucaguucu gg 22
<210> 57
   <211> 99
   <212> RNA
   <213> Bos taurus
<400> 57
   cccaugugua uccucagcuu ugagaacuga auuccauagg uugugucagu gucagaccug      60
   ugaaguuuag uucuuuagcu gggauaucuc uaucauccu                             99
<210> 58
   <211> 24
   <212> RNA
   <213> Bos taurus
<400> 58
   ugagaacuga auuccauagg uugu 24
<210> 59
   <211> 106
   <212> RNA
   <213> Bos taurus
<400> 59
   uaagagaacu uuggccaccu ggcucugaga acugaauucc auaggcugug agcucuagca      60
   aaugcccuag ggacucaguu cuggugcccg gcugugcuac accauc                    106
<210> 60
   <211> 24
   <212> RNA
   <213> Bos taurus
<400> 60
   ugagaacuga auuccauagg cugu 24
<210> 61
   <211> 84
   <212> RNA
   <213> Homo sapiens
<400> 61
   cuccccaugg cccugucucc caacccuugu accagugcug ggcucagacc cugguacagg      60
   ccugggggac agggaccugg ggac                                             84
<210> 62
   <211> 22
   <212> RNA
   <213> Homo sapiens
<400> 62
   ucucccaacc cuuguaccag ug 22
<210> 63
   <211> 100
   <212> RNA
   <213> Bos taurus
<400> 63
   ccucucuccu cacggcccug ucucccaacc cuuguaccag ugugugucuc agacccuggu      60
   acagguacgg ggaggcaggg accuggggga ucccagcagc                            100
<210> 64
   <211> 23
   <212> RNA
   <213> Bos taurus
<400> 64
   ucucccaacc cuuguaccag ugu 23
<210> 65
   <211> 23
   <212> RNA
   <213> Rattus norvegicus
<400> 65
   caaagugcuu acagugcagg uag 23
<210> 66
   <211> 21
   <212> RNA
   <213> Rattus norvegicus
<400> 66
   uauugcacuu gucccggccu g 21
<210> 67
   <211> 22
   <212> RNA
   <213> Rattus norvegicus
<400> 67
   ugagaacuga auuccauggg uu 22
<210> 68
   <211> 22
   <212> RNA
   <213> Homo sapiens
<400> 68
   uagcagcacg uaaauauugg cg 22
<210> 69
   <211> 22
   <212> RNA
   <213> Rattus norvegicus
<400> 69
   uagcagcacg uaaauauugg cg 22
<210> 70
   <211> 22
   <212> RNA
   <213> Bos taurus
<400> 70
   uagcagcacg uaaauauugg ug 22
<210> 71
   <211> 23
   <212> RNA
   <213> Homo sapiens
<400> 71
   uaaggugcau cuagugcaga uag 23
<210> 72
   <211> 22
   <212> RNA
   <213> Bos taurus
<400> 72
   uaaggugcau cuagugcaga ua 22
<210> 73
   <211> 23
   <212> RNA
   <213> Rattus norvegicus
<400> 73
   uaaggugcau cuagugcaga uag 23
<210> 74
   <211> 23
   <212> RNA
   <213> Homo sapiens
<400> 74
   ugugcaaauc caugcaaaac uga 23
<210> 75
   <211> 23
   <212> RNA
   <213> Bos taurus
<400> 75
   ugugcaaauc caugcaaaac uga 23
<210> 76
   <211> 23
   <212> RNA
   <213> Rattus norvegicus
<400> 76
   ugugcaaauc caugcaaaac uga 23
<210> 77
   <211> 23
   <212> RNA
   <213> Homo sapiens
<400> 77
   uaaagugcuu auagugcagg uag 23
<210> 78
   <211> 23
   <212> RNA
   <213> Bos taurus
<400> 78
   uaaagugcuu auagugcagg uag 23
<210> 79
   <211> 23
   <212> RNA
   <213> Rattus norvegicus
<400> 79
   uaaagugcuu auagugcagg uag 23
<210> 80
   <211> 22
   <212> RNA
   <213> Homo sapiens
<400> 80
   uagcuuauca gacugauguu ga 22
<210> 81
   <211> 24
   <212> RNA
   <213> Bos taurus
<400> 81
   uagcuuauca gacugauguu gacu 24
<210> 82
   <211> 22
   <212> RNA
   <213> Rattus norvegicus
<400> 82
   uagcuuauca gacugauguu ga 22
<210> 83
   <211> 21
   <212> RNA
   <213> Homo sapiens
<400> 83
   aucacauugc cagggauuuc c 21
<210> 84
   <211> 22
   <212> RNA
   <213> Bos taurus
<400> 84
   aucacauugc cagggauuuc ca 22
<210> 85
   <211> 21
   <212> RNA
   <213> Rattus norvegicus
<400> 85
   aucacauugc cagggauuuc c 21
<210> 86
   <211> 21
   <212> RNA
   <213> Homo sapiens
<400> 86
   uucacagugg cuaaguuccg c 21
<210> 87
   <211> 21
   <212> RNA
   <213> Rattus norvegicus
<400> 87
   uucacagugg cuaaguuccg c 21
<210> 88
   <211> 20
   <212> RNA
   <213> Bos taurus
<400> 88
   uucacagugg cuaaguuccg 20
<210> 89
   <211> 22
   <212> RNA
   <213> Bos taurus
<400> 89
   agggcuuagc ugcuugugag ca 22
<210> 90
   <211> 21
   <212> RNA
   <213> Homo sapiens
<400> 90
   uucacagugg cuaaguucug c 21
<210> 91
   <211> 21
   <212> RNA
   <213> Bos taurus
<400> 91
   uucacagugg cuaaguucugc 21
<210> 92
   <211> 21
   <212> RNA
   <213> Rattus norvegicus
<400> 92
   uucacagugg cuaaguucug c 21
<210> 93
   <211> 22
   <212> RNA
   <213> Homo sapiens
<400> 93
   uagcaccauc ugaaaucggu ua 22
<210> 94
   <211> 23
   <212> RNA
   <213> Bos taurus
<400> 94
   cuagcaccau cugaaaucgg uua 23
<210> 95
   <211> 22
   <212> RNA
   <213> Rattus norvegicus
<400> 95
   uagcaccauc ugaaaucggu ua 22
<210> 96
   <211> 23
   <212> RNA
   <213> Homo sapiens
<400> 96
   uagcaccauu ugaaaucagu guu 23
<210> 97
   <211> 23
   <212> RNA
   <213> Bos taurus
<400> 97
   uagcaccauu ugaaaucagu guu 23
<210> 98
   <211> 23
   <212> RNA
   <213> Rattus norvegicus
<400> 98
   uagcaccauu ugaaaucagu guu 23
<210> 99
   <211> 22
   <212> RNA
   <213> Homo sapiens
<400> 99
   uagcaccauu ugaaaucggu ua 22
<210> 100
   <211> 22
   <212> RNA
   <213> Bos taurus
<400> 100
   uagcaccauu ugaaaucggu ua 22
<210> 101
   <211> 22
   <212> RNA
   <213> Rattus norvegicus
<400> 101
   uagcaccauu ugaaaucggu ua 22
<210> 102
   <211> 22
   <212> RNA
   <213> Homo sapiens
<400> 102
   ugaccgauuu cuccuggugu uc 22
<210> 103
   <211> 22
   <212> RNA
   <213> Rattus norvegicus
<400> 103
   ugaccgauuu cuccuggugu uc 22
<210> 104
   <211> 22
   <212> RNA
   <213> Homo sapiens
<400> 104
   uguaaacauc cucgacugga ag 22
<210> 105
   <211> 24
   <212> RNA
   <213> Bos taurus
<400> 105
   uguaaacauc cucgacugga agcu 24
<210> 106
   <211> 22
   <212> RNA
   <213> Rattus norvegicus
<400> 106
   uguaaacauc cucgacugga ag 22
<210> 107
   <211> 23
   <212> RNA
   <213> Homo sapiens
<400> 107
   uguaaacauc cuacacucuc agc 23
<210> 108
   <211> 23
   <212> RNA
   <213> Bos taurus
<400> 108
   uguaaacauc cuacacucuc agc 23
<210> 109
   <211> 23
   <212> RNA
   <213> Rattus norvegicus
<400> 109
   uguaaacauc cuacacucuc agc 23
<210> 110
   <211> 22
   <212> RNA
   <213> Homo sapiens
<400> 110
   uguaaacauc cccgacugga ag 22
<210> 111
   <211> 24
   <212> RNA
   <213> Bos taurus
<400> 111
   uguaaacauc cccgacugga agcu 24
<210> 112
   <211> 22
   <212> RNA
   <213> Rattus norvegicus
<400> 112
   uguaaacauc cccgacugga ag 22
<210> 113
   <211> 22
   <212> RNA
   <213> Homo sapiens
<400> 113
   cuuucagucg gauguuuaca gc 22
<210> 114
   <211> 22
   <212> RNA
   <213> Rattus norvegicus
<400> 114
   cuuucagucg gauguuuaca gc 22
<210> 115
   <211> 21
   <212> RNA
   <213> Homo sapiens
<400> 115
   gugcauugua guugcauugc a 21
<210> 116
   <211> 21
   <212> RNA
   <213> Bos taurus
<400> 116
   gugcauugua guugcauugc a 21
<210> 117
   <211> 21
   <212> RNA
   <213> Rattus norvegicus
<400> 117
   gugcauugua guugcauugc a 21
<210> 118
   <211> 22
   <212> RNA
   <213> Homo sapiens
<400> 118
   caaucacuaa cuccacugcc au 22
<210> 119
   <211> 22
   <212> RNA
   <213> Bos taurus
<400> 119
   aggcagugua auuagcugau ug 22
<210> 120
   <211> 23
   <212> RNA
   <213> Rattus norvegicus
<400> 120
   uaggcagugu aauuagcuga uug 23
<210> 121
   <211> 23
   <212> RNA
   <213> Homo sapiens
<400> 121
   caaagugcug uucgugcagg uag 23
<210> 122
   <211> 22
   <212> RNA
   <213> Bos taurus
<400> 122
   caaagugcug uucgugcagg ua 22
<210> 123
   <211> 23
   <212> RNA
   <213> Rattus norvegicus
<400> 123
   caaagugcug uucgugcagg uag 23
<210> 124
   <211> 22
   <212> RNA
   <213> Homo sapiens
<400> 124
   aacccguaga uccgaacuug ug 22
<210> 125
   <211> 22
   <212> RNA
   <213> Bos taurus
<400> 125
   aacccguaga uccgaacuug ug 22
<210> 126
   <211> 22
   <212> RNA
   <213> Rattus norvegicus
<400> 126
   aacccguaga uccgaacuug ug 22
<210> 127
   <211> 21
   <212> RNA
   <213> Homo sapiens
<400> 127
   uacaguacug ugauaacuga a 21
<210> 128
   <211> 21
   <212> RNA
   <213> Bos taurus
<400> 128
   uacaguacug ugauaacuga a 21
<210> 129
   <211> 21
   <212> RNA
   <213> Rattus norvegicus
<400> 129
   uacaguacug ugauaacuga a 21
<210> 130
   <211> 21
   <212> RNA
   <213> Rattus norvegicus
<400> 130
   uacaguacug ugauagcuga a 21
<210> 131
   <211> 21
   <212> RNA
   <213> Bos taurus
<400> 131
   uaaagugcug acagugcaga u 21
<210> 132
   <211> 21
   <212> RNA
   <213> Rattus norvegicus
<400> 132
   uaaagugcug acagugcaga u 21
<210> 133
   <211> 22
   <212> RNA
   <213> Homo sapiens
<400> 133
   cagugcaaug augaaagggc au 22
<210> 134
   <211> 22
   <212> RNA
   <213> Bos taurus
<400> 134
   cagugcaaug augaaaggg cau 22
<210> 135
   <211> 22
   <212> RNA
   <213> Rattus norvegicus
<400> 135
   cagugcaaug augaaagggc au 22
<210> 136
   <211> 21
   <212> RNA
   <213> Homo sapiens
<400> 136
   uaccacaggg uagaaccacg g 21
<210> 137
   <211> 21
   <212> RNA
   <213> Rattus norvegicus
<400> 137
   uaccacaggg uagaaccacg g 21
<210> 138
   <211> 22
   <212> RNA
   <213> Homo sapiens
<400> 138
   uaacacuguc ugguaaagau gg 22
<210> 139
   <211> 22
   <212> RNA
   <213> Bos taurus
<400> 139
   uaacacuguc ugguaaagau gg 22
<210> 140
   <211> 22
   <212> RNA
   <213> Rattus norvegicus
<400> 140
   uaacacuguc ugguaaagau gg 22
<210> 141
   <211> 21
   <212> RNA
   <213> Homo sapiens
<400> 141
   ugagaugaag cacuguagcu c 21
<210> 142
   <211> 22
   <212> RNA
   <213> Bos taurus
<400> 142
   ugagaugaag cacuguagcu cg 22
<210> 143
   <211> 22
   <212> RNA
   <213> Rattus norvegicus
<400> 143
   ugagaugaag cacuguagcu ca 22
<210> 144
   <211> 22
   <212> RNA
   <213> Homo sapiens
<400> 144
   uggagagaaa ggcaguuccu ga 22
<210> 145
   <211> 22
   <212> RNA
   <213> Bos taurus
<400> 145
   uggagagaaa ggcaguuccu ga 22
<210> 146
   <211> 22
   <212> RNA
   <213> Rattus norvegicus
<400> 146
   uggagagaaa ggcaguuccu ga 22
<210> 147
   <211> 22
   <212> RNA
   <213> Homo sapiens
<400> 147
   caaagaauuc uccuuuuggg cu 22
<210> 148
   <211> 22
   <212> RNA
   <213> Bos taurus
<400> 148
   caaagaauuc uccuuuuggg cu 22
<210> 149
   <211> 22
   <212> RNA
   <213> Rattus norvegicus
<400> 149
   caaagaauuc uccuuuuggg cu 22
<210> 150
   <211> 21
   <212> RNA
   <213> Homo sapiens
<400> 150
   cugaccuaug aauugacagc c 21
<210> 151
   <211> 23
   <212> RNA
   <213> Bos taurus
<400> 151
   cugaccuaug aauugacagc cag 23
<210> 152
   <211> 21
   <212> RNA
   <213> Rattus norvegicus
<400> 152
   cugaccuaug aauugacagc c 21
<210> 153
   <211> 22
   <212> RNA
   <213> Homo sapiens
<400> 153
   aacuggccua caaaguccca gu 22
<210> 154
   <211> 22
   <212> RNA
   <213> Bos taurus
<400> 154
   aacuggccua caaaguccca gu 22
<210> 155
   <211> 22
   <212> RNA
   <213> Rattus norvegicus
<400> 155
   aacuggccua caaaguccca gu 22
<210> 156
   <211> 21
   <212> RNA
   <213> Homo sapiens
<400> 156
   uagcagcaca gaaauauugg c 21
<210> 157
   <211> 22
   <212> RNA
   <213> Bos taurus
<400> 157
   uagcagcaca gaaauauugg ca 22
<210> 158
   <211> 21
   <212> RNA
   <213> Rattus norvegicus
<400> 158
   uagcagcaca gaaauauugg c 21
<210> 159
   <211> 22
   <212> RNA
   <213> Homo sapiens
<400> 159
   uaacacuguc ugguaacgau gu 22
<210> 160
   <211> 23
   <212> RNA
   <213> Bos taurus
<400> 160
   uaacacuguc ugguaacgau guu 23
<210> 161
   <211> 22
   <212> RNA
   <213> Rattus norvegicus
<400> 161
   uaacacuguc ugguaacgau gu 22
<210> 162
   <211> 22
   <212> RNA
   <213> Homo sapiens
<400> 162
   uccuucauuc caccggaguc ug 22
<210> 163
   <211> 22
   <212> RNA
   <213> Bos taurus
<400> 163
   uccuucauuc caccggaguc ug 22
<210> 164
   <211> 22
   <212> RNA
   <213> Rattus norvegicus
<400> 164
   uccuucauuc caccggaguc ug 22
<210> 165
   <211> 21
   <212> RNA
   <213> Homo sapiens
<400> 165
   uugugcuuga ucuaaccaug u 21
<210> 166
   <211> 21
   <212> RNA
   <213> Rattus norvegicus
<400> 166
   uugugcuuga ucuaaccaug u 21
<210> 167
   <211> 21
   <212> RNA
   <213> Homo sapiens
<400> 167
   ugauugucca aacgcaauuc u 21
<210> 168
   <211> 21
   <212> RNA
   <213> Rattus norvegicus
<400> 168
   ugauugucca aacgcaauuc u 21
<210> 169
   <211> 23
   <212> RNA
   <213> Homo sapiens
<400> 169
   agcuacauug ucugcugggu uuc 23
<210> 170
   <211> 22
   <212> RNA
   <213> Bos taurus
<400> 170
   agcuacauug ucugcugggu uu 22
<210> 171
   <211> 23
   <212> RNA
   <213> Rattus norvegicus
<400> 171
   agcuacauug ucugcugggu uuc 23
<210> 172
   <211> 23
   <212> RNA
   <213> Homo sapiens
<400> 172
   cagugcaaua guauugucaa agc 23
<210> 173
   <211> 25
   <212> RNA
   <213> Bos taurus
<400> 173
   cagugcaaua guauugucaa agcau 25
<210> 174
   <211> 23
   <212> RNA
   <213> Rattus norvegicus
<400> 174
   cagugcaaua guauugucaa agc 23
<210> 175
   <211> 22
   <212> RNA
   <213> Rattus norvegicus
<400> 175
   cagcagcaau ucauguuuug ga 22
<210> 176
   <211> 22
   <212> RNA
   <213> Homo sapiens
<400> 176
   uuauaaagca augagacuga uu 22
<210> 177
   <211> 23
   <212> RNA
   <213> Bos taurus
<400> 177
   uccgucucag uuacuuuaua gcc 23
<210> 178
   <211> 22
   <212> RNA
   <213> Rattus norvegicus
<400> 178
   uuauaaagca augagacuga uu 22
<210> 179
   <211> 22
   <212> RNA
   <213> Homo sapiens
<400> 179
   uuaucagaau cuccaggggu ac 22
<210> 180
   <211> 22
   <212> RNA
   <213> Bos taurus
<400> 180
   uuaucagaau cuccaggggu ac 22
<210> 181
   <211> 22
   <212> RNA
   <213> Rattus norvegicus
<400> 181
   uuaucagaau cuccaggggu ac 22
<210> 182
   <211> 22
   <212> RNA
   <213> Homo sapiens
<400> 182
   uaauacuguc ugguaaaacc gu 22
<210> 183
   <211> 22
   <212> RNA
   <213> Bos taurus
<400> 183
   uaauacuguc ugguaaugcc gu 22
<210> 184
   <211> 22
   <212> RNA
   <213> Rattus norvegicus
<400> 184
   uaauacuguc ugguaaugcc gu 22
<210> 185
   <211> 22
   <212> RNA
   <213> Homo sapiens
<400> 185
   uaugugccuu uggacuacau cg 22
<210> 186
   <211> 21
   <212> RNA
   <213> Bos taurus
<400> 186
   uaugugccuu uggacuacau c 21
<210> 187
   <211> 22
   <212> RNA
   <213> Rattus norvegicus
<400> 187
   uaugugccuu uggacuacau cg 22
<210> 188
   <211> 22
   <212> RNA
   <213> Rattus norvegicus
<400> 188
   uaugugugug uguaugucca ug 22
<210> 189
   <211> 21
   <212> RNA
   <213> Homo sapiens
<400> 189
   cagcagcaca cugugguuug u 21
<210> 190
   <211> 22
   <212> RNA
   <213> Bos taurus
<400> 190
   cagcagcaca cugugguuug ua 22
<210> 191
   <211> 22
   <212> RNA
   <213> Rattus norvegicus
<400> 191
   cagcagcaca cugugguuug ua 22
<210> 192
   <211> 23
   <212> RNA
   <213> Homo sapiens
<400> 192
   uaauccuugc uaccugggug aga 23
<210> 193
   <211> 23
   <212> RNA
   <213> Bos taurus
<400> 193
   uaauccuugc uaccugggug aga 23
<210> 194
   <211> 22
   <212> RNA
   <213> Rattus norvegicus
<400> 194
   aaugcaccug ggcaaggguu ca 22
<210> 195
   <211> 23
   <212> RNA
   <213> Homo sapiens
<400> 195
   uagcagcggg aacaguucug cag 23
<210> 196
   <211> 23
   <212> RNA
   <213> Rattus norvegicus
<400> 196
   uagcagcggg aacaguacug cag 23
<210> 197
   <211> 22
   <212> RNA
   <213> Homo sapiens
<400> 197
   caugccuuga guguaggacc gu 22
<210> 198
   <211> 22
   <212> RNA
   <213> Bos taurus
<400> 198
   caugccuuga guguaggacc gu 22
<210> 199
   <211> 22
   <212> RNA
   <213> Rattus norvegicus
<400> 199
   caugccuuga guguaggacu gu 22
<210> 200
   <211> 22
   <212> RNA
   <213> Homo sapiens
<400> 200
   ugugacagau ugauaacuga aa 22
<210> 201
   <211> 22
   <212> RNA
   <213> Rattus norvegicus
<400> 201
   ugugacagau ugauaacuga aa 22
<210> 202
   <211> 22
   <212> RNA
   <213> Homo sapiens
<400> 202
   ugagguagua gguuguauag uu 22
<210> 203
   <211> 22
   <212> RNA
   <213> Bos taurus
<400> 203
   ugagguagua gguuguauag uu 22
<210> 204
   <211> 22
   <212> RNA
   <213> Rattus norvegicus
<400> 204
   ugagguagua gguuguauag uu 22
<210> 205
   <211> 21
   <212> RNA
   <213> Homo sapiens
<400> 205
   cuauacaauc uacugucuuu c 21
<210> 206
   <211> 21
   <212> RNA
   <213> Bos taurus
<400> 206
   cuauacaauc uacugucuuu c 21
<210> 207
   <211> 22
   <212> RNA
   <213> Rattus norvegicus
<400> 207
   ugagguagua gguuguauag uu 22
<210> 208
   <211> 22
   <212> RNA
   <213> Homo sapiens
<400> 208
   ugagguagua gguugugugg uu 22
<210> 209
   <211> 22
   <212> RNA
   <213> Bos taurus
<400> 209
   ugagguagua gguugugugg uu 22
<210> 210
   <211> 22
   <212> RNA
   <213> Rattus norvegicus
<400> 210
   ugagguagua gguugugugg uu 22
<210> 211
   <211> 22
   <212> RNA
   <213> Homo sapiens
<400> 211
   ugagguagua gguuguaugg uu 22
<210> 212
   <211> 22
   <212> RNA
   <213> Bos taurus
<400> 212
   ugagguagua gguuguaugg uu 22
<210> 213
   <211> 22
   <212> RNA
   <213> Rattus norvegicus
<400> 213
   ugagguagua gguuguaugg uu 22
<210> 214
   <211> 22
   <212> RNA
   <213> Homo sapiens
<400> 214
   agagguagua gguugcauag uu 22
<210> 215
   <211> 22
   <212> RNA
   <213> Bos taurus
<400> 215
   agagguagua gguugcauag uu 22
<210> 216
   <211> 22
   <212> RNA
   <213> Rattus norvegicus
<400> 216
   agagguagua gguugcauag uu 22
<210> 217
   <211> 22
   <212> RNA
   <213> Homo sapiens
<400> 217
   ugagguagga gguuguauag uu 22
<210> 218
   <211> 21
   <212> RNA
   <213> Bos taurus
<400> 218
   ugagguagga gguuguauag u 21
<210> 219
   <211> 22
   <212> RNA
   <213> Rattus norvegicus
<400> 219
   ugagguagga gguuguauag uu 22
<210> 220
   <211> 22
   <212> RNA
   <213> Homo sapiens
<400> 220
   ugagguagua gauuguauag uu 22
<210> 221
   <211> 22
   <212> RNA
   <213> Bos taurus
<400> 221
   ugagguagua gauuguauag uu 22
<210> 222
   <211> 22
   <212> RNA
   <213> Rattus norvegicus
<400> 222
   ugagguagua gauuguauag uu 22
<210> 223
   <211> 22
   <212> RNA
   <213> Rattus norvegicus
<400> 223
   ugagguagua guuugugcug uu 22
<210> 224
   <211> 23
   <212> RNA
   <213> Homo sapiens
<400> 224
   uacccuguag auccgaauuu gug 23
<210> 225
   <211> 23
   <212> RNA
   <213> Bos taurus
<400> 225
   uacccuguag auccgaauuu gug 23
<210> 226
   <211> 23
   <212> RNA
   <213> Rattus norvegicus
<400> 226
   uacccuguag auccgaauuu gug 23
<210> 227
   <211> 23
   <212> RNA
   <213> Homo sapiens
<400> 227
   uacccuguag aaccgaauuu gug 23
<210> 228
   <211> 23
   <212> RNA
   <213> Bos taurus
<400> 228
   uacccuguag aaccgaauuu gug 23
<210> 229
   <211> 22
   <212> RNA
   <213> Rattus norvegicus
<400> 229
   cccuguagaa ccgaauuugu gu 22
<210> 230
   <211> 22
   <212> RNA
   <213> Homo sapiens
<400> 230
   uagcagcaca ucaugguuua ca 22
<210> 231
   <211> 22
   <212> RNA
   <213> Bos taurus
<400> 231
   uagcagcaca ucaugguuua ca 22
<210> 232
   <211> 22
   <212> RNA
   <213> Rattus norvegicus
<400> 232
   uagcagcaca ucaugguuua ca 22
<210> 233
   <211> 23
   <212> RNA
   <213> Homo sapiens
<400> 233
   ugugcaaauc uaugcaaaac uga 23
<210> 234
   <211> 23
   <212> RNA
   <213> Bos taurus
<400> 234
   ugugcaaauc uaugcaaaac uga 23
<210> 235
   <211> 23
   <212> RNA
   <213> Rattus norvegicus
<400> 235
   ugugcaaauc uaugcaaaac uga 23
<210> 236
   <211> 22
   <212> RNA
   <213> Homo sapiens
<400> 236
   acugcauuau gagcacuuaa ag 22
<210> 237
   <211> 21
   <212> RNA
   <213> Rattus norvegicus
<400> 237
   acugcauuac gagcacuuac a 21
<210> 238
   <211> 22
   <212> RNA
   <213> Homo sapiens
<400> 238
   aagcugccag uugaagaacu gu 22
<210> 239
   <211> 21
   <212> RNA
   <213> Bos taurus
<400> 239
   aagcugccag uugaagaacu g 21
<210> 240
   <211> 22
   <212> RNA
   <213> Rattus norvegicus
<400> 240
   aagcugccag uugaagaacu gu 22
<210> 241
   <211> 21
   <212> RNA
   <213> Homo sapiens
<400> 241
   aucacauugc cagggauuac c 21
<210> 242
   <211> 21
   <212> RNA
   <213> Rattus norvegicus
<400> 242
   aucacauugc cagggauuac c 21
<210> 243
   <211> 21
   <212> RNA
   <213> Bos taurus
<400> 243
   ggguuccugg caugcugauu u 21
<210> 244
   <211> 23
   <212> RNA
   <213> Bos taurus
<400> 244
   aucacauugc cagggauuac cac 23
<210> 245
   <211> 22
   <212> RNA
   <213> Homo sapiens
<400> 245
   uggcucaguu cagcaggaac ag 22
<210> 246
   <211> 23
   <212> RNA
   <213> Bos taurus
<400> 246
   gugccuacug agcugauauc agu 23
<210> 247
   <211> 22
   <212> RNA
   <213> Rattus norvegicus
<400> 247
   uggcucaguu cagcaggaac ag 22
<210> 248
   <211> 22
   <212> RNA
   <213> Homo sapiens
<400> 248
   cauugcacuu gucucggucu ga 22
<210> 249
   <211> 22
   <212> RNA
   <213> Bos taurus
<400> 249
   cauugcacuu gucucggucu ga 22
<210> 250
   <211> 22
   <212> RNA
   <213> Rattus norvegicus
<400> 250
   cauugcacuu gucucggucu ga 22
<210> 251
   <211> 22
   <212> RNA
   <213> Homo sapiens
<400> 251
   uucaaguaau ccaggauagg cu 22
<210> 252
   <211> 22
   <212> RNA
   <213> Bos taurus
<400> 252
   uucaaguaau ccaggauagg cu 22
<210> 253
   <211> 22
   <212> RNA
   <213> Rattus norvegicus
<400> 253
   uucaaguaau ccaggauagg cu 22
<210> 254
   <211> 21
   <212> RNA
   <213> Homo sapiens
<400> 254
   uucaaguaau ucaggauagg u 21
<210> 255
   <211> 21
   <212> RNA
   <213> Rattus norvegicus
<400> 255
   uucaaguaau ucaggauagg u 21
<210> 256
   <211> 22
   <212> RNA
   <213> Homo sapiens
<400> 256
   aaggagcuca cagucuauug ag 22
<210> 257
   <211> 22
   <212> RNA
   <213> Bos taurus
<400> 257
   aaggagcuca cagucuauug ag 22
<210> 258
   <211> 22
   <212> RNA
   <213> Rattus norvegicus
<400> 258
   aaggagcuca cagucuauug ag 22
<210> 259
   <211> 22
   <212> RNA
   <213> Homo sapiens
<400> 259
   cuuucagucg gauguuugca gc 22
<210> 260
   <211> 22
   <212> RNA
   <213> Rattus norvegicus
<400> 260
   cuuucagucg gauguuugca gc 22
<210> 261
   <211> 22
   <212> RNA
   <213> Homo sapiens
<400> 261
   uguaaacauc cuacacucag cu 22
<210> 262
   <211> 22
   <212> RNA
   <213> Bos taurus
<400> 262
   uguaaacauc cuacacucag cu 22
<210> 263
   <211> 22
   <212> RNA
   <213> Rattus norvegicus
<400> 263
   uguaaacauc cuacacucag cu 22
<210> 264
   <211> 22
   <212> RNA
   <213> Homo sapiens
<400> 264
   cugggagagg guuguuuacu cc 22
<210> 265
   <211> 22
   <212> RNA
   <213> Rattus norvegicus
<400> 265
   cugggagagg guuguuuacu cc 22
<210> 266
   <211> 22
   <212> RNA
   <213> Homo sapiens
<400> 266
   cugggagaag gcuguuuacu cu 22
<210> 267
   <211> 22
   <212> RNA
   <213> Rattus norvegicus
<400> 267
   cugggagaag gcuguuuacu cu 22
<210> 268
   <211> 22
   <212> RNA
   <213> Homo sapiens
<400> 268
   uguaaacauc cuugacugga ag 22
<210> 269
   <211> 24
   <212> RNA
   <213> Bos taurus
<400> 269
   uguaaacauc cuugacugga agcu 24
<210> 270
   <211> 22
   <212> RNA
   <213> Rattus norvegicus
<400> 270
   uguaaacauc cuugacugga ag 22
<210> 271
   <211> 21
   <212> RNA
   <213> Homo sapiens
<400> 271
   aggcaagaug cuggcauagc u 21
<210> 272
   <211> 21
   <212> RNA
   <213> Bos taurus
<400> 272
   aggcaagaug cuggcauagc u 21
<210> 273
   <211> 22
   <212> RNA
   <213> Rattus norvegicus
<400> 273
   aggcaagaug cuggcauagc ug 22
<210> 274
   <211> 22
   <212> RNA
   <213> Homo sapiens
<400> 274
   uggcaguguc uuagcugguu gu 22
<210> 275
   <211> 22
   <212> RNA
   <213> Bos taurus
<400> 275
   uggcaguguc uuagcugguu gu 22
<210> 276
   <211> 22
   <212> RNA
   <213> Rattus norvegicus
<400> 276
   uggcaguguc uuagcugguu gu 22
<210> 277
   <211> 23
   <212> RNA
   <213> Homo sapiens
<400> 277
   uuuggcacua gcacauuuuu gcu 23
<210> 278
   <211> 23
   <212> RNA
   <213> Bos taurus
<400> 278
   uuuggcacua gcacauuuuu gcu 23
<210> 279
   <211> 23
   <212> RNA
   <213> Rattus norvegicus
<400> 279
   uuuggcacua gcacauuuuu gcu 23
<210> 280
   <211> 22
   <212> RNA
   <213> Homo sapiens
<400> 280
   ugagguagua aguuguauug uu 22
<210> 281
   <211> 22
   <212> RNA
   <213> Bos taurus
<400> 281
   ugagguagua aguuguauug uu 22
<210> 282
   <211> 22
   <212> RNA
   <213> Rattus norvegicus
<400> 282
   ugagguagua aguuguauug uu 22
<210> 283
   <211> 22
   <212> RNA
   <213> Homo sapiens
<400> 283
   aacccguaga uccgaucuug ug 22
<210> 284
   <211> 21
   <212> RNA
   <213> Bos taurus
<400> 284
   aacccguaga uccgaucuug u 21
<210> 285
   <211> 22
   <212> RNA
   <213> Rattus norvegicus
<400> 285
   aacccguaga uccgaucuug ug 22
<210> 286
   <211> 22
   <212> RNA
   <213> Homo sapiens
<400> 286
   cacccguaga accgaccuug cg 22
<210> 287
   <211> 22
   <212> RNA
   <213> Bos taurus
<400> 287
   cacccguaga accgaccuug cg 22
<210> 288
   <211> 22
   <212> RNA
   <213> Rattus norvegicus
<400> 288
   cacccguaga accgaccuug cg 22
<210> 289
   <211> 23
   <212> RNA
   <213> Homo sapiens
<400> 289
   agcagcauug uacagggcua uga 23
<210> 290
   <211> 23
   <212> RNA
   <213> Bos taurus
<400> 290
   agcagcauug uacagggcua uga 23
<210> 291
   <211> 23
   <212> RNA
   <213> Rattus norvegicus
<400> 291
   agcagcauug uacagggcua uga 23
<210> 292
   <211> 23
   <212> RNA
   <213> Homo sapiens
<400> 292
   agcagcauug uacagggcua uca 23
<210> 293
   <211> 22
   <212> RNA
   <213> Bos taurus
<400> 293
   agcagcauug uacagggcua uc 22
<210> 294
   <211> 23
   <212> RNA
   <213> Rattus norvegicus
<400> 294
   agcagcauug uacagggcua uca 23
<210> 295
   <211> 22
   <212> RNA
   <213> Rattus norvegicus
<400> 295
   acaggugagg uucuugggag cc 22
<210> 296
   <211> 24
   <212> RNA
   <213> Rattus norvegicus
<400> 296
   ucccugagac ccuuuaaccu guga 24
<210> 297
   <211> 22
   <212> RNA
   <213> Rattus norvegicus
<400> 297
   ucccugagac ccuaacuugu ga 22
<210> 298
   <211> 22
   <212> RNA
   <213> Homo sapiens
<400> 298
   acggguuagg cucuugggag cu 22
<210> 299
   <211> 22
   <212> RNA
   <213> Rattus norvegicus
<400> 299
   acggguuagg cucuugggag cu 22
<210> 300
   <211> 21
   <212> RNA
   <213> Homo sapiens
<400> 300
   ucacagugaa ccggucucuu u 21
<210> 301
   <211> 21
   <212> RNA
   <213> Bos taurus
<400> 301
   ucacagugaa ccggucucuu u 21
<210> 302
   <211> 21
   <212> RNA
   <213> Rattus norvegicus
<400> 302
   ucacagugaa ccggucucuu u 21
<210> 303
   <211> 22
   <212> RNA
   <213> Homo sapiens
<400> 303
   cagugcaaug uuaaaagggc au 22
<210> 304
   <211> 22
   <212> RNA
   <213> Bos taurus
<400> 304
   cagugcaaug uuaaaagggc au 22
<210> 305
   <211> 22
   <212> RNA
   <213> Rattus norvegicus
<400> 305
   cagugcaaug uuaaaagggc au 22
<210> 306
   <211> 22
   <212> RNA
   <213> Homo sapiens
<400> 306
   uuuggucccc uucaaccagc ug 22
<210> 307
   <211> 22
   <212> RNA
   <213> Bos taurus
<400> 307
   uuuggucccc uucaaccagc ug 22
<210> 308
   <211> 22
   <212> RNA
   <213> Rattus norvegicus
<400> 308
   uuuggucccc uucaaccagc ug 22
<210> 309
   <211> 22
   <212> RNA
   <213> Homo sapiens
<400> 309
   uuuggucccc uucaaccagc ua 22
<210> 310
   <211> 22
   <212> RNA
   <213> Bos taurus
<400> 310
   uuuggucccc uucaaccagc ua 22
<210> 311
   <211> 22
   <212> RNA
   <213> Rattus norvegicus
<400> 311
   uuuggucccc uucaaccagc ua 22
<210> 312
   <211> 22
   <212> RNA
   <213> Homo sapiens
<400> 312
   ugugacuggu ugaccagagg gg 22
<210> 313
   <211> 22
   <212> RNA
   <213> Bos taurus
<400> 313
   ugugacuggu ugaccagagu gg 22
<210> 314
   <211> 22
   <212> RNA
   <213> Rattus norvegicus
<400> 314
   ugugacuggu ugaccagagg gg 22
<210> 315
   <211> 22
   <212> RNA
   <213> Homo sapiens
<400> 315
   ggagacgcgg cccuguugga gu 22
<210> 316
   <211> 22
   <212> RNA
   <213> Rattus norvegicus
<400> 316
   uggagacgcg gcccuguugg ag 22
<210> 317
   <211> 22
   <212> RNA
   <213> Homo sapiens
<400> 317
   cagugguuuu acccuauggu ag 22
<210> 318
   <211> 22
   <212> RNA
   <213> Bos taurus
<400> 318
   uaccacaggg uagaaccacg ga 22
<210> 319
   <211> 22
   <212> RNA
   <213> Rattus norvegicus
<400> 319
   cagugguuuu acccuauggu ag 22
<210> 320
   <211> 24
   <212> RNA
   <213> Rattus norvegicus
<400> 320
   ugagaacuga auuccauagg cugu 24
<210> 321
   <211> 22
   <212> RNA
   <213> Homo sapiens
<400> 321
   ucagugcauc acagaacuuu gu 22
<210> 322
   <211> 22
   <212> RNA
   <213> Bos taurus
<400> 322
   ucagugcauc acagaacuuu gu 22
<210> 323
   <211> 22
   <212> RNA
   <213> Rattus norvegicus
<400> 323
   ucagugcauc acagaacuuu gu 22
<210> 324
   <211> 21
   <212> RNA
   <213> Homo sapiens
<400> 324
   ucgaggagcu cacagucuag u 21
<210> 325
   <211> 21
   <212> RNA
   <213> Bos taurus
<400> 325
   cuagacugaa gcuccuugag g 21
<210> 326
   <211> 21
   <212> RNA
   <213> Rattus norvegicus
<400> 326
   cuagacugaa gcuccuugag g 21
<210> 327
   <211> 21
   <212> RNA
   <213> Homo sapiens
<400> 327
   ucagugcaug acagaacuug g 21
<210> 328
   <211> 22
   <212> RNA
   <213> Bos taurus
<400> 328
   ucagugcaug acagaacuug gg 22
<210> 329
   <211> 21
   <212> RNA
   <213> Rattus norvegicus
<400> 329
   ucagugcaug acagaacuug g 21
<210> 330
   <211> 23
   <212> RNA
   <213> Rattus norvegicus
<400> 330
   aacauucaac gcugucggug agu 23
<210> 331
   <211> 22
   <212> RNA
   <213> Homo sapiens
<400> 331
   accaucgacc guugauugua cc 22
<210> 332
   <211> 22
   <212> RNA
   <213> Rattus norvegicus
<400> 332
   accaucgacc guugauugua cc 22
<210> 333
   <211> 23
   <212> RNA
   <213> Rattus norvegicus
<400> 333
   aacauucauu gcugucggug ggu 23
<210> 334
   <211> 22
   <212> RNA
   <213> Homo sapiens
<400> 334
   aacauucaac cugucgguga gu 22
<210> 335
   <211> 24
   <212> RNA
   <213> Bos taurus
<400> 335
   aacauucaac cugucgguga guuu 24
<210> 336
   <211> 22
   <212> RNA
   <213> Rattus norvegicus
<400> 336
   aacauucaac cugucgguga gu 22
<210> 337
   <211> 23
   <212> RNA
   <213> Homo sapiens
<400> 337
   aacauucauu guugucggug ggu 23
<210> 338
   <211> 23
   <212> RNA
   <213> Bos taurus
<400> 338
   aacauucauu guugucggug ggu 23
<210> 339
   <211> 23
   <212> RNA
   <213> Rattus norvegicus
<400> 339
   aacauucauu guugucggug ggu 23
<210> 340
   <211> 24
   <212> RNA
   <213> Homo sapiens
<400> 340
   uuuggcaaug guagaacuca cacu 24
<210> 341
   <211> 24
   <212> RNA
   <213> Bos taurus
<400> 341
   uuuggcaaug guagaacuca cacu 24
<210> 342
   <211> 25
   <212> RNA
   <213> Rattus norvegicus
<400> 342
   uuuggcaaug guagaacuca caccg 25
<210> 343
   <211> 22
   <212> RNA
   <213> Homo sapiens
<400> 343
   uauggcacug guagaauuca cu 22
<210> 344
   <211> 23
   <212> RNA
   <213> Bos taurus
<400> 344
   uauggcacug guagaauuca cug 23
<210> 345
   <211> 22
   <212> RNA
   <213> Rattus norvegicus
<400> 345
   uauggcacug guagaauuca cu 22
<210> 346
   <211> 21
   <212> RNA
   <213> Homo sapiens
<400> 346
   caucccuugc augguggagg g 21
<210> 347
   <211> 22
   <212> RNA
   <213> Bos taurus
<400> 347
   caucccuugc augguggagg gu 22
<210> 348
   <211> 21
   <212> RNA
   <213> Rattus norvegicus
<400> 348
   caucccuugc augguggagg g 21
<210> 349
   <211> 22
   <212> RNA
   <213> Rattus norvegicus
<400> 349
   uagguaguuu cguguuguug gg 22
<210> 350
   <211> 22
   <212> RNA
   <213> Homo sapiens
<400> 350
   acaguagucu gcacauuggu ua 22
<210> 351
   <211> 22
   <212> RNA
   <213> Bos taurus
<400> 351
   acaguagucu gcacauuggu ua 22
<210> 352
   <211> 22
   <212> RNA
   <213> Rattus norvegicus
<400> 352
   acaguagucu gcacauuggu ua 22
<210> 353
   <211> 22
   <212> RNA
   <213> Homo sapiens
<400> 353
   uaauacugcc ugguaaugau ga 22
<210> 354
   <211> 21
   <212> RNA
   <213> Bos taurus
<400> 354
   uaauacugcc ugguaaugau g 21
<210> 355
   <211> 23
   <212> RNA
   <213> Rattus norvegicus
<400> 355
   uaauacugcc ugguaaugau gac 23
<210> 356
   <211> 23
   <212> RNA
   <213> Homo sapiens
<400> 356
   uaauacugcc ggguaaugau gga 23
<210> 357
   <211> 23
   <212> RNA
   <213> Bos taurus
<400> 357
   uaauacugcc ggguaaugau gga 23
<210> 358
   <211> 22
   <212> RNA
   <213> Rattus norvegicus
<400> 358
   uaauacugcc ggguaaugau gg 22
<210> 359
   <211> 22
   <212> RNA
   <213> Homo sapiens
<400> 359
   gugaaauguu uaggaccacu ag 22
<210> 360
   <211> 22
   <212> RNA
   <213> Rattus norvegicus
<400> 360
   gugaaauguu uaggaccacu ag 22
<210> 361
   <211> 22
   <212> RNA
   <213> Homo sapiens
<400> 361
   uucccuuugu cauccuaugc cu 22
<210> 362
   <211> 22
   <212> RNA
   <213> Bos taurus
<400> 362
   uucccuuugu cauccuaugc cu 22
<210> 363
   <211> 22
   <212> RNA
   <213> Rattus norvegicus
<400> 363
   uucccuuugu cauccuaugc cu 22
<210> 364
   <211> 22
   <212> RNA
   <213> Homo sapiens
<400> 364
   uggaauguaa ggaagugugu gg 22
<210> 365
   <211> 22
   <212> RNA
   <213> Bos taurus
<400> 365
   uggaauguaa ggaagugugu gg 22
<210> 366
   <211> 22
   <212> RNA
   <213> Rattus norvegicus
<400> 366
   uggaauguaa ggaagugugu gg 22
<210> 367
   <211> 22
   <212> RNA
   <213> Homo sapiens
<400> 367
   cugugcgugu gacagcggcu ga 22
<210> 368
   <211> 23
   <212> RNA
   <213> Bos taurus
<400> 368
   acugugcgug ugacagcggc uga 23
<210> 369
   <211> 22
   <212> RNA
   <213> Rattus norvegicus
<400> 369
   cugugcgugu gacagcggcu ga 22
<210> 370
   <211> 21
   <212> RNA
   <213> Homo sapiens
<400> 370
   uaacagucuc cagucacggc c 21
<210> 371
   <211> 23
   <212> RNA
   <213> Bos taurus
<400> 371
   accuuggcuc uagacugcuu acu 23
<210> 372
   <211> 22
   <212> RNA
   <213> Rattus norvegicus
<400> 372
   uaacagucuc cagucacggc ca 22
<210> 373
   <211> 22
   <212> RNA
   <213> Homo sapiens
<400> 373
   acagcaggca cagacaggca gu 22
<210> 374
   <211> 22
   <212> RNA
   <213> Bos taurus
<400> 374
   acagcaggca cagacaggca gu 22
<210> 375
   <211> 21
   <212> RNA
   <213> Rattus norvegicus
<400> 375
   acagcaggca cagacaggca g 21
<210> 376
   <211> 21
   <212> RNA
   <213> Homo sapiens
<400> 376
   agcuacaucu ggcuacuggg u 21
<210> 377
   <211> 21
   <212> RNA
   <213> Bos taurus
<400> 377
   agcuacaucu ggcuacuggg u 21
<210> 378
   <211> 21
   <212> RNA
   <213> Rattus norvegicus
<400> 378
   agcuacaucu ggcuacuggg u 21
<210> 379
   <211> 21
   <212> RNA
   <213> Rattus norvegicus
<400> 379
   ugucaguuug ucaaauaccc c 21
<210> 380
   <211> 23
   <212> RNA
   <213> Rattus norvegicus
<400> 380
   cucaaacuau gggggcacuu uuu 23
<210> 381
   <211> 22
   <212> RNA
   <213> Rattus norvegicus
<400> 381
   caucaaagug gaggcccucu cu 22
<210> 382
   <211> 22
   <212> RNA
   <213> Rattus norvegicus
<400> 382
   acucaaacug ggggcucuuu ug 22
<210> 383
   <211> 21
   <212> RNA
   <213> Rattus norvegicus
<400> 383
   cucaaaaugg aggcccuauc u 21
<210> 384
   <211> 21
   <212> RNA
   <213> Homo sapiens
<400> 384
   agggcccccc cucaauccug u 21
<210> 385
   <211> 21
   <212> RNA
   <213> Rattus norvegicus
<400> 385
   agggcccccc cucaauccug u 21
<210> 386
   <211> 22
   <212> RNA
   <213> Homo sapiens
<400> 386
   aaaagcuggg uugagagggc ga 22
<210> 387
   <211> 22
   <212> RNA
   <213> Bos taurus
<400> 387
   aaaagcuggg uugagagggc ga 22
<210> 388
   <211> 22
   <212> RNA
   <213> Rattus norvegicus
<400> 388
   aaaagcuggg uugagagggc ga 22
<210> 389
   <211> 20
   <212> RNA
   <213> Homo sapiens
<400> 389
   acugccccag gugcugcugg 20
<210> 390
   <211> 22
   <212> RNA
   <213> Rattus norvegicus
<400> 390
   ccacugcccc aggugcugcu gg 22
<210> 391
   <211> 23
   <212> RNA
   <213> Homo sapiens
<400> 391
   cgcauccccu agggcauugg ugu 23
<210> 392
   <211> 23
   <212> RNA
   <213> Bos taurus
<400> 392
   cgcauccccu agggcauugg ugu 23
<210> 393
   <211> 23
   <212> RNA
   <213> Rattus norvegicus
<400> 393
   cgcauccccu agggcauugg ugu 23
<210> 394
   <211> 19
   <212> RNA
   <213> Rattus norvegicus
<400> 394
   ccuugagggg caugagggu 19
<210> 395
   <211> 22
   <212> RNA
   <213> Homo sapiens
<400> 395
   cuggcccucu cugcccuucc gu 22
<210> 396
   <211> 22
   <212> RNA
   <213> Bos taurus
<400> 396
   cuggcccucu cugcccuucc gu 22
<210> 397
   <211> 22
   <212> RNA
   <213> Rattus norvegicus
<400> 397
   cuggcccucu cugcccuucc gu 22
<210> 398
   <211> 21
   <212> RNA
   <213> Homo sapiens
<400> 398
   gccccugggc cuauccuaga a 21
<210> 399
   <211> 21
   <212> RNA
   <213> Bos taurus
<400> 399
   gccccugggc cuauccuaga a 21
<210> 400
   <211> 21
   <212> RNA
   <213> Rattus norvegicus
<400> 400
   gccccugggc cuauccuaga a 21
<210> 401
   <211> 22
   <212> RNA
   <213> Homo sapiens
<400> 401
   acucggcgug gcgucggucg ug 22
<210> 402
   <211> 22
   <212> RNA
   <213> Bos taurus
<400> 402
   acucggcgug gcgucggucg ug 22
<210> 403
   <211> 23
   <212> RNA
   <213> Rattus norvegicus
<400> 403
   uccgucucag uuacuuuaua gcc 23
<210> 404
   <211> 21
   <212> RNA
   <213> Rattus norvegicus
<400> 404
   ucggucgauc ggucggucgg u 21
<210> 405
   <211> 23
   <212> RNA
   <213> Homo sapiens
<400> 405
   ucucacacag aaaucgcacc cgu 23
<210> 406
   <211> 25
   <212> RNA
   <213> Bos taurus
<400> 406
   ucucacacag aaaucgcacc caucu 25
<210> 407
   <211> 23
   <212> RNA
   <213> Rattus norvegicus
<400> 407
   ucucacacag aaaucgcacc cgu 23
<210> 408
   <211> 22
   <212> RNA
   <213> Homo sapiens
<400> 408
   gcugacuccu aguccagggc uc 22
<210> 409
   <211> 21
   <212> RNA
   <213> Bos taurus
<400> 409
   gcugacuccu aguccagugc u 21
<210> 410
   <211> 21
   <212> RNA
   <213> Rattus norvegicus
<400> 410
   ugcugacccc uaguccagug c 21
<210> 411
   <211> 19
   <212> RNA
   <213> Rattus norvegicus
<400> 411
   ugucccucug ggucgccca 19
<210> 412
   <211> 21
   <212> RNA
   <213> Rattus norvegicus
<400> 412
   agaguaguag guugcauagu a 21
<210> 413
   <211> 22
   <212> RNA
   <213> Homo sapiens
<400> 413
   uaaugccccu aaaaauccuu au 22
<210> 414
   <211> 22
   <212> RNA
   <213> Bos taurus
<400> 414
   uaaugccccu aaaaauccuu au 22
<210> 415
   <211> 22
   <212> RNA
   <213> Rattus norvegicus
<400> 415
   uaaugccccu aaaaauccuu au 22
<210> 416
   <211> 22
   <212> RNA
   <213> Homo sapiens
<400> 416
   gccugcuggg guggaaccug gu 22
<210> 417
   <211> 22
   <212> RNA
   <213> Bos taurus
<400> 417
   gccugcuggg guggaaccug gu 22
<210> 418
   <211> 23
   <212> RNA
   <213> Rattus norvegicus
<400> 418
   gccugcuggg guggaaccug guu 23
<210> 419
   <211> 22
   <212> RNA
   <213> Homo sapiens
<400> 419
   uuuguucguu cggcucgcgu ga 22
<210> 420
   <211> 23
   <212> RNA
   <213> Bos taurus
<400> 420
   uuuuguucgu ucggcucgcg uga 23
<210> 421
   <211> 22
   <212> RNA
   <213> Rattus norvegicus
<400> 421
   uuuguucguu cggcucgcgu ga 22
<210> 422
   <211> 21
   <212> RNA
   <213> Homo sapiens
<400> 422
   acuggacuug gagucagaag g 21
<210> 423
   <211> 22
   <212> RNA
   <213> Bos taurus
<400> 423
   acuggacuug gagucagaag gc 22
<210> 424
   <211> 21
   <212> RNA
   <213> Rattus norvegicus
<400> 424
   acuggacuug gagucagaag g 21
<210> 425
   <211> 22
   <212> RNA
   <213> Homo sapiens
<400> 425
   cuccugacuc cagguccugu gu 22
<210> 426
   <211> 22
   <212> RNA
   <213> Rattus norvegicus
<400> 426
   cuccugacuc cagguccugu gu 22
<210> 427
   <211> 23
   <212> RNA
   <213> Homo sapiens
<400> 427
   aaugacacga ucacucccgu uga 23
<210> 428
   <211> 22
   <212> RNA
   <213> Bos taurus
<400> 428
   augacacgau cacucccguu ga 22
<210> 429
   <211> 23
   <212> RNA
   <213> Rattus norvegicus
<400> 429
   aaugacacga ucacucccgu uga 23
<210> 430
   <211> 22
   <212> RNA
   <213> Rattus norvegicus
<400> 430
   uauuuagaac ggugcuggug ug 22
<210> 431
   <211> 21
   <212> RNA
   <213> Homo sapiens
<400> 431
   ucacuccucu ccucccgucu u 21
<210> 432
   <211> 21
   <212> RNA
   <213> Bos taurus
<400> 432
   ucacuccucu ccucccgucu u 21
<210> 433
   <211> 23
   <212> RNA
   <213> Rattus norvegicus
<400> 433
   ucacuccucc ccucccgucu ugu 23
<210> 434
   <211> 22
   <212> RNA
   <213> Homo sapiens
<400> 434
   ucaggcucag uccccucccg au 22
<210> 435
   <211> 22
   <212> RNA
   <213> Bos taurus
<400> 435
   ucaggcucag uccccucccg au 22
<210> 436
   <211> 22
   <212> RNA
   <213> Rattus norvegicus
<400> 436
   ucaggcucag uccccucccg au 22
<210> 437
   <211> 22
   <212> RNA
   <213> Homo sapiens
<400> 437
   ugaaacauac acgggaaacc uc 22
<210> 438
   <211> 22
   <212> RNA
   <213> Bos taurus
<400> 438
   ugaaacauac acgggaaacc uc 22
<210> 439
   <211> 21
   <212> RNA
   <213> Rattus norvegicus
<400> 439
   ugaaacauac acgggaaacc u 21
<210> 440
   <211> 23
   <212> RNA
   <213> Homo sapiens
<400> 440
   ucggggauca ucaugucacg aga 23
<210> 441
   <211> 22
   <212> RNA
   <213> Bos taurus
<400> 441
   ucggggauca ucaugucacg ag 22
<210> 442
   <211> 22
   <212> RNA
   <213> Rattus norvegicus
<400> 442
   cucggggauc aucaugucac ga 22
<210> 443
   <211> 21
   <212> RNA
   <213> Homo sapiens
<400> 443
   aauggcgcca cuaggguugu g 21
<210> 444
   <211> 21
   <212> RNA
   <213> Rattus norvegicus
<400> 444
   aauggcgcca cuaggguugu g 21
<210> 445
   <211> 23
   <212> RNA
   <213> Homo sapiens
<400> 445
   ugagguuggu guacugugug uga 23
<210> 446
   <211> 23
   <212> RNA
   <213> Rattus norvegicus
<400> 446
   ugagguuggu guacugugug uga 23
<210> 447
   <211> 21
   <212> RNA
   <213> Bos taurus
<400> 447
   ucaauggcug aggugaggua c 21
<210> 448
   <211> 21
   <212> RNA
   <213> Rattus norvegicus
<400> 448
   ucaauggcug aggugaggca c 21
<210> 449
   <211> 20
   <212> RNA
   <213> Homo sapiens
<400> 449
   cggcucuggg ucugugggga 20
<210> 450
   <211> 22
   <212> RNA
   <213> Bos taurus
<400> 450
   ccccucaguc caccagagcc cg 22
<210> 451
   <211> 20
   <212> RNA
   <213> Rattus norvegicus
<400> 451
   cggcucuggg ucugugggga 20
<210> 452
   <211> 21
   <212> RNA
   <213> Homo sapiens
<400> 452
   aagguuacuu guuaguucag g 21
<210> 453
   <211> 21
   <212> RNA
   <213> Rattus norvegicus
<400> 453
   aagguuacuu guuaguucag g 21
<210> 454
   <211> 22
   <212> RNA
   <213> Homo sapiens
<400> 454
   cugcccuggc ccgagggacc ga 22
<210> 455
   <211> 22
   <212> RNA
   <213> Bos taurus
<400> 455
   cugcccuggc ccgagggacc ga 22
<210> 456
   <211> 22
   <212> RNA
   <213> Rattus norvegicus
<400> 456
   cugcccuggc ccgagggacc ga 22
<210> 457
   <211> 19
   <212> RNA
   <213> Homo sapiens
<400> 457
   gugaggacuc gggaggugg 19
<210> 458
   <211> 21
   <212> RNA
   <213> Bos taurus
<400> 458
   gugaggacuc gggaggugga g 21
<210> 459
   <211> 21
   <212> RNA
   <213> Rattus norvegicus
<400> 459
   gugaggacug gggaggugga g 21
<210> 460
   <211> 22
   <212> RNA
   <213> Rattus norvegicus
<400> 460
   ugggucuuug cgggcaagau ga 22
<210> 461
   <211> 22
   <212> RNA
   <213> Homo sapiens
<400> 461
   ugggucuuug cgggcgagau ga 22
<210> 462
   <211> 22
   <212> RNA
   <213> Bos taurus
<400> 462
   ugggucuuug cgggcgagau ga 22
<210> 463
   <211> 22
   <212> RNA
   <213> Homo sapiens
<400> 463
   gaauguugcu cggugaaccc cu 22
<210> 464
   <211> 20
   <212> RNA
   <213> Rattus norvegicus
<400> 464
   aauguugcuc ggugaacccc 20
<210> 465
   <211> 23
   <212> RNA
   <213> Bos taurus
<400> 465
   agguuacccg agcaacuuug cau 23
<210> 466
   <211> 23
   <212> RNA
   <213> Homo sapiens
<400> 466
   uauucauuua uccccagccu aca 23
<210> 467
   <211> 22
   <212> RNA
   <213> Bos taurus
<400> 467
   caggcugggg ugugugugga ug 22
<210> 468
   <211> 22
   <212> RNA
   <213> Rattus norvegicus
<400> 468
   uauucauuua cuccccagcc ua 22
<210> 469
   <211> 20
   <212> RNA
   <213> Homo sapiens
<400> 469
   guagaggaga uggcgcaggg 20
<210> 470
   <211> 20
   <212> RNA
   <213> Bos taurus
<400> 470
   guagaggaga uggcgcaggg 20
<210> 471
   <211> 20
   <212> RNA
   <213> Rattus norvegicus
<400> 471
   guagaggaga uggcgcaggg 20
<210> 472
   <211> 22
   <212> RNA
   <213> Bos taurus
<400> 472
   uucaaguaau ucaggauagg uu 22
<210> 473
   <211> 22
   <212> RNA
   <213> Homo sapiens
<400> 473
   uagcagcaca uaaugguuug ug 22
<210> 474
   <211> 21
   <212> RNA
   <213> Bos taurus
<400> 474
   uagcagcaca uaaugguuug u 21
<210> 475
   <211> 21
   <212> RNA
   <213> Bos taurus
<400> 475
   uagcagcacg uaaauauugg c 21
<210> 476
   <211> 24
   <212> RNA
   <213> Bos taurus
<400> 476
   uguaaacacc cuacacucuc agcu 24
<210> 477
   <211> 22
   <212> RNA
   <213> Bos taurus
<400> 477
   aaaagugcuu acagugcagg ua 22
<210> 478
   <211> 22
   <212> RNA
   <213> Homo sapiens
<400> 478
   ucguaccgug aguaauaaug cg 22
<210> 479
   <211> 21
   <212> RNA
   <213> Bos taurus
<400> 479
   cguaccguga guaauaaugc g 21
<210> 480
   <211> 22
   <212> RNA
   <213> Rattus norvegicus
<400> 480
   ucguaccgug aguaauaaug cg 22
<210> 481
   <211> 22
   <212> RNA
   <213> Homo sapiens
<400> 481
   aagcccuuac cccaaaaagc au 22
<210> 482
   <211> 22
   <212> RNA
   <213> Bos taurus
<400> 482
   aagcccuuac cccaaaaagc au 22
<210> 483
   <211> 22
   <212> RNA
   <213> Homo sapiens
<400> 483
   uggacggaga acugauaagg gu 22
<210> 484
   <211> 22
   <212> RNA
   <213> Bos taurus
<400> 484
   uggacggaga acugauaagg gu 22
<210> 485
   <211> 22
   <212> RNA
   <213> Rattus norvegicus
<400> 485
   uggacggaga acugauaagg gu 22
<210> 486
   <211> 22
   <212> RNA
   <213> Homo sapiens
<400> 486
   uagguaguuu cauguuguug gg 22
<210> 487
   <211> 22
   <212> RNA
   <213> Bos taurus
<400> 487
   uagguaguuu cauguuguug gg 22
<210> 488
   <211> 22
   <212> RNA
   <213> Rattus norvegicus
<400> 488
   uagguaguuu cauguuguug gg 22
<210> 489
   <211> 22
   <212> RNA
   <213> Homo sapiens
<400> 489
   uccagcauca gugauuuugu ug 22
<210> 490
   <211> 23
   <212> RNA
   <213> Bos taurus
<400> 490
   uccagcauca gugauuuugu uga 23
<210> 491
   <211> 23
   <212> RNA
   <213> Rattus norvegicus
<400> 491
   uccagcauca gugauuuugu uga 23
<210> 492
   <211> 24
   <212> RNA
   <213> Homo sapiens
<400> 492
   aauccuugga accuaggugu gagu 24
<210> 493
   <211> 24
   <212> RNA
   <213> Bos taurus
<400> 493
   aauccuugga accuaggugu gagu 24
<210> 494
   <211> 24
   <212> RNA
   <213> Rattus norvegicus
<400> 494
   aauccuugga accuaggugu gaau 24
<210> 495
   <211> 22
   <212> RNA
   <213> Homo sapiens
<400> 495
   aacuguuugc agaggaaacu ga 22
<210> 496
   <211> 22
   <212> RNA
   <213> Bos taurus
<400> 496
   uguuugcaga ggaaacugag ac 22
<210> 497
   <211> 22
   <212> RNA
   <213> Homo sapiens
<400> 497
   uccuguacug agcugccccg ag 22
<210> 498
   <211> 22
   <212> RNA
   <213> Bos taurus
<400> 498
   uccuguacug agcugccccg ag 22
<210> 499
   <211> 22
   <212> RNA
   <213> Homo sapiens
<400> 499
   uuaugguuug ccugggacug ag 22
<210> 500
   <211> 19
   <212> RNA
   <213> Bos taurus
<400> 500
   ugguuugccu gggacugag 19
<210> 501
   <211> 23
   <212> RNA
   <213> Homo sapiens
<400> 501
   aaggagcuua caaucuagcu ggg 23
<210> 502
   <211> 23
   <212> RNA
   <213> Bos taurus
<400> 502
   aaggagcuua caaucuagcu ggg 23
<210> 503
   <211> 23
   <212> RNA
   <213> Rattus norvegicus
<400> 503
   aaggagcuua caaucuagcu ggg 23
<210> 504
   <211> 18
   <212> RNA
   <213> Bos taurus
<400> 504
   ucgagaagga ggcugcug 18